# EUROPEAN PATENT APPLICATION

(11) **EP 1 264 884 A1**
(43) Date of publication of application: **11.12.2002**
(21) Application number: 01904423.9
(22) Date of filing: 14.02.2001
(51) Int. Cl.: C12N 15/09, A61K 35/76, A61K 38/00, A61K 48/00, A61P 35/00, A61P 43/00, C07H 21/00, C07K 14/395, C12N 9/16

(54) **METHOD OF CONTROLLING TELOMERE LENGTH**

(30) Priority: 18.02.2000 JP 2000041929
(71) Applicant: Riken, Wako-shi, Saitama 351-0198 (JP); JAPAN SCIENCE AND TECHNOLOGY CORPORATION, Kawaguchi-shi, Saitama 332-0012 (JP)
(72) Inventor: OHTA, Kunihiro, Nerima-ku, Tokyo 179-0072 (JP); SHIBATA, Takehiko, Shinjuku-ku, Tokyo 161-0033 (JP)
(74) Representative: Maschio, Antonio
(86) International application number: JP0101024
(87) International publication number: WO01060996

(57) **Abstract**

A method of controlling telomere length wherein the method comprises introducing into a cell a DNA encoding Mrell protein or a DNA encoding a protein comprising Mrell protein wherein a part of the nuclease domain, the C-terminal domain or the whole thereof is modified or deleted.

SEQUENCE LISTING

<110> RIKEN ; Japan Science and Technology Corporation

<120> METHOD OF CONTROLLING TELOMERE LENGTH

<130> PH-1136-PCT

<150> JP 2000-41929
<151> 2000-2-18

<160> 13

<210> 1
<211> 2079
<212> DNA
<213> Saccharomyces cerevisiae

<220>
<221> CDS
<222> (1)..(2076)

<400> 1
atg gac tat cct gat cca gac aca ata agg att tta att act aca gat 48
Met Asp Tyr Pro Asp Pro Asp Thr Ile Arg Ile Leu Ile Thr Thr Asp
1               5                   10                  15
aat cat gtg ggt tac aac gaa aat gat ccc att act ggc gat gat tct 96
Asn His Val Gly Tyr Asn Glu Asn Asp Pro Ile Thr Gly Asp Asp Ser
            20                  25                  30

tgg aaa act ttc cat gaa gtc atg atg ctg gcc aaa aat aac aac gta 144
Trp Lys Thr Phe His Glu Val Met Met Leu Ala Lys Asn Asn Asn Val
        35                  40                  45
gac atg gtt gta cag tcc ggt gat ctt ttt cac gtg aat aag cct tcc 192
Asp Met Val Val Gln Ser Gly Asp Leu Phe His Val Asn Lys Pro Ser
    50                  55                  60
aag aag tca ctc tac caa gta ctg aaa act ttg aga tta tgt tgc atg 240
Lys Lys Ser Leu Tyr Gln Val Leu Lys Thr Leu Arg Leu Cys Cys Met
65                  70                  75                  80
ggt gac aag cct tgc gag tta gaa tta ttg agc gat ccc tca caa gtt 288
Gly Asp Lys Pro Cys Glu Leu Glu Leu Leu Ser Asp Pro Ser Gln Val
                85                  90                  95
ttt cac tac gat gaa ttt acc aac gtt aac tat gag gac ccc aac ttt 336
Phe His Tyr Asp Glu Phe Thr Asn Val Asn Tyr Glu Asp Pro Asn Phe
            100                 105                 110
aat att tct att ccc gta ttc ggc ata tca ggt aat cat gat gat gcg 384
Asn Ile Ser Ile Pro Val Phe Gly Ile Ser Gly Asn His Asp Asp Ala
        115                 120                 125
tcg ggg gac tca ctg ttg tgt cct atg gat ata ctt cat gcg act ggt 432
Ser Gly Asp Ser Leu Leu Cys Pro Met Asp Ile Leu His Ala Thr Gly
    130                 135                 140
cta ata aat cat ttc ggg aaa gtc atc gaa tct gat aaa ata aaa gtc 480
Leu Ile Asn His Phe Gly Lys Val Ile Glu Ser Asp Lys Ile Lys Val
145                 150                 155                 160
gtg cca tta tta ttt cag aaa ggg tcc act aag tta gca ttg tac gga 528
Val Pro Leu Leu Phe Gln Lys Gly Ser Thr Lys Leu Ala Leu Tyr Gly
                165                 170                 175
tta gcc gct gtt cgt gat gaa agg tta ttt aga act ttt aag gat ggt 576
Leu Ala Ala Val Arg Asp Glu Arg Leu Phe Arg Thr Phe Lys Asp Gly

            180                 185                 190
ggt gtc act ttt gaa gta ccg act atg cga gaa ggt gaa tgg ttt aat 624
Gly Val Thr Phe Glu Val Pro Thr Met Arg Glu Gly Glu Trp Phe Asn
        195                 200                 205
tta atg tgc gtc cat caa aat cat aca ggt cac acg aat act gca ttt 672
Leu Met Cys Val His Gln Asn His Thr Gly His Thr Asn Thr Ala Phe
    210                 215                 220
tta cct gaa cag ttc ttg cca gat ttc ctg gat atg gtg ata tgg ggt 720
Leu Pro Glu Gln Phe Leu Pro Asp Phe Leu Asp Met Val Ile Trp Gly
225                 230                 235                 240
cat gaa cat gag tgt att ccg aat ctc gta cac aat cca att aaa aat 768
His Glu His Glu Cys Ile Pro Asn Leu Val His Asn Pro Ile Lys Asn
                245                 250                 255
ttt gat gta tta caa cct ggt tca tct gta gct act tca ctt tgt gag 816
Phe Asp Val Leu Gln Pro Gly Ser Ser Val Ala Thr Ser Leu Cys Glu
            260                 265                 270
gct gag gca caa ccc aag tat gtc ttc atc ctt gac ata aag tat gga 864
Ala Glu Ala Gln Pro Lys Tyr Val Phe Ile Leu Asp Ile Lys Tyr Gly
        275                 280                 285
gaa gca cca aaa atg aca cct att cct ctt gag act ata cgg aca ttc 912
Glu Ala Pro Lys Met Thr Pro Ile Pro Leu Glu Thr Ile Arg Thr Phe
    290                 295                 300
aaa atg aaa tcc att tcg tta caa gat gtt ccc cat ttg agg cct cac 960
Lys Met Lys Ser Ile Ser Leu Gln Asp Val Pro His Leu Arg Pro His
305                 310                 315                 320
gat aaa gat gct acg tct aag tat ctt att gaa caa gtt gaa gaa atg 1008
Asp Lys Asp Ala Thr Ser Lys Tyr Leu Ile Glu Gln Val Glu Glu Met
                325                 330                 335
atc cgc gac gct aat gag gaa act aaa caa aaa tta gcg gac gat ggt 1056

Ile Arg Asp Ala Asn Glu Glu Thr Lys Gln Lys Leu Ala Asp Asp Gly
            340                 345                 350
gaa ggt gac atg gtt gcg gaa tta ccg aaa cca ttg atc aga tta cgt 1104
Glu Gly Asp Met Val Ala Glu Leu Pro Lys Pro Leu Ile Arg Leu Arg
        355                 360                 365
gtt gat tat agt gca ccc tcc aat aca caa tcc tca ata gat tac caa 1152
Val Asp Tyr Ser Ala Pro Ser Asn Thr Gln Ser Ser Ile Asp Tyr Gln
    370                 375                 380
gta gaa aac ccg cgt aga ttt agc aat cga ttt gtg gga cgt gtt gct 1200
Val Glu Asn Pro Arg Arg Phe Ser Asn Arg Phe Val Gly Arg Val Ala
385                 390                 395                 400
aac ggt aat aac gtt gtg cag ttt tat aaa aaa agg tca cct gta act 1248
Asn Gly Asn Asn Val Val Gln Phe Tyr Lys Lys Arg Ser Pro Val Thr
                405                 410                 415
aga tca aaa aaa tcc ggt ata aat gga aca agc atc agt gat aga gat 1296
Arg Ser Lys Lys Ser Gly Ile Asn Gly Thr Ser Ile Ser Asp Arg Asp
            420                 425                 430
gtt gag aaa ctt ttc agc gaa agt ggc ggt gaa cta gaa gtt caa act 1344
Val Glu Lys Leu Phe Ser Glu Ser Gly Gly Glu Leu Glu Val Gln Thr
        435                 440                 445
ttg gtt aat gat ctc ttg aac aaa atg caa cta tct tta tta cca gaa 1392
Leu Val Asn Asp Leu Leu Asn Lys Met Gln Leu Ser Leu Leu Pro Glu
    450                 455                 460
gtt ggt ttg aat gaa gca gta aag aag ttt gta gat aaa gat gag aaa 1440
Val Gly Leu Asn Glu Ala Val Lys Lys Phe Val Asp Lys Asp Glu Lys
465                 470                 475                 480
aca gct ctt aaa gaa ttt att agc cat gaa ata tcg aac gaa gtt gga 1488
Thr Ala Leu Lys Glu Phe Ile Ser His Glu Ile Ser Asn Glu Val Gly
                485                 490                 495

ata tta tct acg aat gaa gaa ttt ctg aga aca gat gat gca gag gaa 1536
Ile Leu Ser Thr Asn Glu Glu Phe Leu Arg Thr Asp Asp Ala Glu Glu
            500                 505                 510
atg aaa gcg ctt ata aaa cag gtt aag cgt gct aac agt gtt agg ccg 1584
Met Lys Ala Leu Ile Lys Gln Val Lys Arg Ala Asn Ser Val Arg Pro
        515                 520                 525
act ccc cct aaa gaa aat gat gag aca aat ttc gca ttc aat ggt aat 1632
Thr Pro Pro Lys Glu Asn Asp Glu Thr Asn Phe Ala Phe Asn Gly Asn
    530                 535                 540
ggg cta gat tcc ttc cgg tct agt aat aga gaa gta aga act gga tct 1680
Gly Leu Asp Ser Phe Arg Ser Ser Asn Arg Glu Val Arg Thr Gly Ser
545                 550                 555                 560
cca gac att acc caa tca cat gtt gat aat gaa tca aga ata acc cat 1728
Pro Asp Ile Thr Gln Ser His Val Asp Asn Glu Ser Arg Ile Thr His
                565                 570                 575
att agt caa gcg gaa agc agt aag cca acg agc aaa ccc aaa cga gtg 1776
Ile Ser Gln Ala Glu Ser Ser Lys Pro Thr Ser Lys Pro Lys Arg Val
            580                 585                 590
cga act gca acg aaa aag aaa att cct gct ttt tca gac tca act gtc 1824
Arg Thr Ala Thr Lys Lys Lys Ile Pro Ala Phe Ser Asp Ser Thr Val
        595                 600                 605
ata tcc gat gca gaa aat gaa ctc ggt gat aat aac gat gct caa gat 1872
Ile Ser Asp Ala Glu Asn Glu Leu Gly Asp Asn Asn Asp Ala Gln Asp
    610                 615                 620
gat gtt gat att gat gag aat gac ata att atg gtc agt act gac gaa 1920
Asp Val Asp Ile Asp Glu Asn Asp Ile Ile Met Val Ser Thr Asp Glu
625                 630                 635                 640
gag gac gct agt tat ggt tta ctt aat ggt cga aaa aca aaa aca aag 1968
Glu Asp Ala Ser Tyr Gly Leu Leu Asn Gly Arg Lys Thr Lys Thr Lys

                645                 650                 655
act cgt tct gct gcg agc acc aaa acc gct tcc aga agg gga aaa gga 2016
Thr Arg Ser Ala Ala Ser Thr Lys Thr Ala Ser Arg Arg Gly Lys Gly
            660                 665                 670
aga gca tca agg acg cca aag acg gat att ctt gga agt ctc ctt gct 2064
Arg Ala Ser Arg Thr Pro Lys Thr Asp Ile Leu Gly Ser Leu Leu Ala
        675                 680                 685
aag aaa aga aaa tag 2079
Lys Lys Arg Lys
    690

<210> 2
<211> 692
<212> PRT
<213> Saccharomyces cerevisiae

<400> 2
Met Asp Tyr Pro Asp Pro Asp Thr Ile Arg Ile Leu Ile Thr Thr Asp
1               5                   10                  15
Asn His Val Gly Tyr Asn Glu Asn Asp Pro Ile Thr Gly Asp Asp Ser
            20                  25                  30
Trp Lys Thr Phe His Glu Val Met Met Leu Ala Lys Asn Asn Asn Val
        35                  40                  45
Asp Met Val Val Gln Ser Gly Asp Leu Phe His Val Asn Lys Pro Ser
    50                  55                  60
Lys Lys Ser Leu Tyr Gln Val Leu Lys Thr Leu Arg Leu Cys Cys Met
65                  70                  75                  80
Gly Asp Lys Pro Cys Glu Leu Glu Leu Leu Ser Asp Pro Ser Gln Val
                85                  90                  95

Phe His Tyr Asp Glu Phe Thr Asn Val Asn Tyr Glu Asp Pro Asn Phe
            100                 105                 110
Asn Ile Ser Ile Pro Val Phe Gly Ile Ser Gly Asn His Asp Asp Ala
        115                 120                 125
Ser Gly Asp Ser Leu Leu Cys Pro Met Asp Ile Leu His Ala Thr Gly
    130                 135                 140
Leu Ile Asn His Phe Gly Lys Val Ile Glu Ser Asp Lys Ile Lys Val
145                 150                 155                 160
Val Pro Leu Leu Phe Gln Lys Gly Ser Thr Lys Leu Ala Leu Tyr Gly
                165                 170                 175
Leu Ala Ala Val Arg Asp Glu Arg Leu Phe Arg Thr Phe Lys Asp Gly
            180                 185                 190
Gly Val Thr Phe Glu Val Pro Thr Met Arg Glu Gly Glu Trp Phe Asn
        195                 200                 205
Leu Met Cys Val His Gln Asn His Thr Gly His Thr Asn Thr Ala Phe
    210                 215                 220
Leu Pro Glu Gln Phe Leu Pro Asp Phe Leu Asp Met Val Ile Trp Gly
225                 230                 235                 240
His Glu His Glu Cys Ile Pro Asn Leu Val His Asn Pro Ile Lys Asn
                245                 250                 255
Phe Asp Val Leu Gln Pro Gly Ser Ser Val Ala Thr Ser Leu Cys Glu
            260                 265                 270
Ala Glu Ala Gln Pro Lys Tyr Val Phe Ile Leu Asp Ile Lys Tyr Gly
        275                 280                 285
Glu Ala Pro Lys Met Thr Pro Ile Pro Leu Glu Thr Ile Arg Thr Phe
    290                 295                 300
Lys Met Lys Ser Ile Ser Leu Gln Asp Val Pro His Leu Arg Pro His
305                 310                 315                 320
Asp Lys Asp Ala Thr Ser Lys Tyr Leu Ile Glu Gln Val Glu Glu Met

                325                 330                 335
Ile Arg Asp Ala Asn Glu Glu Thr Lys Gln Lys Leu Ala Asp Asp Gly
            340                 345                 350
Glu Gly Asp Met Val Ala Glu Leu Pro Lys Pro Leu Ile Arg Leu Arg
        355                 360                 365
Val Asp Tyr Ser Ala Pro Ser Asn Thr Gln Ser Ser Ile Asp Tyr Gln
    370                 375                 380
Val Glu Asn Pro Arg Arg Phe Ser Asn Arg Phe Val Gly Arg Val Ala
385                 390                 395                 400
Asn Gly Asn Asn Val Val Gln Phe Tyr Lys Lys Arg Ser Pro Val Thr
                405                 410                 415
Arg Ser Lys Lys Ser Gly Ile Asn Gly Thr Ser Ile Ser Asp Arg Asp
            420                 425                 430
Val Glu Lys Leu Phe Ser Glu Ser Gly Gly Glu Leu Glu Val Gln Thr
        435                 440                 445
Leu Val Asn Asp Leu Leu Asn Lys Met Gln Leu Ser Leu Leu Pro Glu
    450                 455                 460
Val Gly Leu Asn Glu Ala Val Lys Lys Phe Val Asp Lys Asp Glu Lys
465                 470                 475                 480
Thr Ala Leu Lys Glu Phe Ile Ser His Glu Ile Ser Asn Glu Val Gly
                485                 490                 495
Ile Leu Ser Thr Asn Glu Glu Phe Leu Arg Thr Asp Asp Ala Glu Glu
            500                 505                 510
Met Lys Ala Leu Ile Lys Gln Val Lys Arg Ala Asn Ser Val Arg Pro
        515                 520                 525
Thr Pro Pro Lys Glu Asn Asp Glu Thr Asn Phe Ala Phe Asn Gly Asn
    530                 535                 540
Gly Leu Asp Ser Phe Arg Ser Ser Asn Arg Glu Val Arg Thr Gly Ser
545                 550                 555                 560

Pro Asp Ile Thr Gln Ser His Val Asp Asn Glu Ser Arg Ile Thr His
                565                 570                 575
Ile Ser Gln Ala Glu Ser Ser Lys Pro Thr Ser Lys Pro Lys Arg Val
            580                 585                 590
Arg Thr Ala Thr Lys Lys Lys Ile Pro Ala Phe Ser Asp Ser Thr Val
        595                 600                 605
Ile Ser Asp Ala Glu Asn Glu Leu Gly Asp Asn Asn Asp Ala Gln Asp
    610                 615                 620
Asp Val Asp Ile Asp Glu Asn Asp Ile Ile Met Val Ser Thr Asp Glu
625                 630                 635                 640
Glu Asp Ala Ser Tyr Gly Leu Leu Asn Gly Arg Lys Thr Lys Thr Lys
                645                 650                 655
Thr Arg Ser Ala Ala Ser Thr Lys Thr Ala Ser Arg Arg Gly Lys Gly
            660                 665                 670
Arg Ala Ser Arg Thr Pro Lys Thr Asp Ile Leu Gly Ser Leu Leu Ala
        675                 680                 685
Lys Lys Arg Lys
    690

<210> 3
<211> 2319
<212> DNA
<213> Homo sapiens

<220>
<221> CDS
<222> (1)..(2316)

<400> 3

atg tcg gcc acg gga aag gtt tct agg cca att caa gtt ttc acg cgc 48
Met Ser Ala Thr Gly Lys Val Ser Arg Pro Ile Gln Val Phe Thr Arg
1               5                   10                  15
gca aac tat ttt ttc aaa ctc att ttt cag ttt aaa gtc cga aaa tat 96
Ala Asn Tyr Phe Phe Lys Leu Ile Phe Gln Phe Lys Val Arg Lys Tyr
            20                  25                  30
aac tct ctc act gta gaa cgt att ttc ata aga gcc atg tgt ggc agt 144
Asn Ser Leu Thr Val Glu Arg Ile Phe Ile Arg Ala Met Cys Gly Ser
        35                  40                  45
gat gac agt ttt gac gac ttt gta cca gat agt caa gag cca gca tct 192
Asp Asp Ser Phe Asp Asp Phe Val Pro Asp Ser Gln Glu Pro Ala Ser
    50                  55                  60
tcg cgt acc cga aat cag gat cat ctc gac gat gac gag gtt ccc tgc 240
Ser Arg Thr Arg Asn Gln Asp His Leu Asp Asp Asp Glu Val Pro Cys
65                  70                  75                  80
tca caa agg cca gat gct gcc aac gac act atg gtt gaa gat ctc gac 288
Ser Gln Arg Pro Asp Ala Ala Asn Asp Thr Met Val Glu Asp Leu Asp
                85                  90                  95
gag gga gac gag cca gct cat gac gaa tct gag gac atc atc aaa att 336
Glu Gly Asp Glu Pro Ala His Asp Glu Ser Glu Asp Ile Ile Lys Ile
            100                 105                 110
ctc gtt gcc act gat att cat tgc ggt tat gga gaa aat aaa gcc aac 384
Leu Val Ala Thr Asp Ile His Cys Gly Tyr Gly Glu Asn Lys Ala Asn
        115                 120                 125
att cat atg gat gcc gtc aac act ttt gaa gaa gtg ctt caa att gcc 432
Ile His Met Asp Ala Val Asn Thr Phe Glu Glu Val Leu Gln Ile Ala
    130                 135                 140
act gaa caa aaa gtc gat atg att tta ctt gga gga gat ctt ttt cat 480
Thr Glu Gln Lys Val Asp Met Ile Leu Leu Gly Gly Asp Leu Phe His

145                 150                 155                 160

gaa aat aat cca tct aga gaa gtt caa cat cgt gtt aca caa ttg ctc 528

Glu Asn Asn Pro Ser Arg Glu Val Gln His Arg Val Thr Gln Leu Leu

                165                 170                 175

cgt caa tat tgc ttg aat ggg aat cca atc gcg ttg gag ttt ctc tca 576

Arg Gln Tyr Cys Leu Asn Gly Asn Pro Ile Ala Leu Glu Phe Leu Ser

            180                 185                 190

gat gct tct gtc aat ttt aat caa agt gtg ttt ggt cac gtg aac tat 624

Asp Ala Ser Val Asn Phe Asn Gln Ser Val Phe Gly His Val Asn Tyr

        195                 200                 205

tac gat cag aat ttg aat gtc ggt ctt cca att ttc aca att cat gga 672

Tyr Asp Gln Asn Leu Asn Val Gly Leu Pro Ile Phe Thr Ile His Gly

    210                 215                 220

aat cat gat gat cta tca gga aaa gga ctt act gct ctt gat ctt ctt 720

Asn His Asp Asp Leu Ser Gly Lys Gly Leu Thr Ala Leu Asp Leu Leu

225                 230                 235                 240

cac gaa tct gga ctt gta aat ctg ttt gga aag cac agc aac att cag 768

His Glu Ser Gly Leu Val Asn Leu Phe Gly Lys His Ser Asn Ile Gln

                245                 250                 255

gag ttt att gtt tca cca att ttg ctt aga aaa gga gaa act cgt ttg 816

Glu Phe Ile Val Ser Pro Ile Leu Leu Arg Lys Gly Glu Thr Arg Leu

            260                 265                 270

gcg tta tac gga att gga agt caa aga gat gat cgt ctt gtt cga gca 864

Ala Leu Tyr Gly Ile Gly Ser Gln Arg Asp Asp Arg Leu Val Arg Ala

        275                 280                 285

ttc aaa aat aat agt att tca ttt ttg cgg cca aac gct ggc gcc gag 912

Phe Lys Asn Asn Ser Ile Ser Phe Leu Arg Pro Asn Ala Gly Ala Glu

    290                 295                 300

gat tgg ttt aat ttg ttt gtc ctc cat caa aat cgt cca cgg aga gcc 960

Asp Trp Phe Asn Leu Phe Val Leu His Gln Asn Arg Pro Arg Arg Ala
305                 310                 315                 320
atg cac cga tcg aca gga aac ttt ctt ccc gaa tct ctg att cct caa 1008
Met His Arg Ser Thr Gly Asn Phe Leu Pro Glu Ser Leu Ile Pro Gln
                325                 330                 335
ttc ttt gac ctt ctt att tgg gga cac gaa cac gaa tgt aaa cct gat 1056
Phe Phe Asp Leu Leu Ile Trp Gly His Glu His Glu Cys Lys Pro Asp
            340                 345                 350
cct caa tat gtt gct tca tcg gaa gct gtt ggt gat gga ttt tac att 1104
Pro Gln Tyr Val Ala Ser Ser Glu Ala Val Gly Asp Gly Phe Tyr Ile
        355                 360                 365
ctt caa cca gga tcg act gtt gct act tcg ctc act ccg gaa gaa gcc 1152
Leu Gln Pro Gly Ser Thr Val Ala Thr Ser Leu Thr Pro Glu Glu Ala
    370                 375                 380
ctg cag aaa aat gca ttc cta ata aag att aaa gga aga aag ttt gct 1200
Leu Gln Lys Asn Ala Phe Leu Ile Lys Ile Lys Gly Arg Lys Phe Ala
385                 390                 395                 400
tca aag cct att cct ctt caa aca gtt cgt cca atg gtt tgc gat gag 1248
Ser Lys Pro Ile Pro Leu Gln Thr Val Arg Pro Met Val Cys Asp Glu
                405                 410                 415
ctt ctt ctt gat aaa ata cct cca gga agt aga ccg att ttg aaa act 1296
Leu Leu Leu Asp Lys Ile Pro Pro Gly Ser Arg Pro Ile Leu Lys Thr
            420                 425                 430
gat cgt cca aaa cac acg gat ggc cga tac ata gat gaa att gcc att 1344
Asp Arg Pro Lys His Thr Asp Gly Arg Tyr Ile Asp Glu Ile Ala Ile
        435                 440                 445
gaa gcg aaa ata aat gaa atg att aca aca gcg aaa gcc aag aga cga 1392
Glu Ala Lys Ile Asn Glu Met Ile Thr Thr Ala Lys Ala Lys Arg Arg
    450                 455                 460

cca cgg caa cct gaa tta cct ttg att cga ctc aaa gtg atc tat gat     1440
Pro Arg Gln Pro Glu Leu Pro Leu Ile Arg Leu Lys Val Ile Tyr Asp
465                 470                 475                 480
ggt gat tgg ctc aat att act cca gca aat gct aaa aga att ggt ctt     1488
Gly Asp Trp Leu Asn Ile Thr Pro Ala Asn Ala Lys Arg Ile Gly Leu
                485                 490                 495
cgt tat gaa aat gta gtg gca aac gca gtg gat atg gtt ttt atc aag     1536
Arg Tyr Glu Asn Val Val Ala Asn Ala Val Asp Met Val Phe Ile Lys
            500                 505                 510
aaa aat aat aag cca aag gag gga aaa tta caa acc gaa aac gag aaa     1584
Lys Asn Asn Lys Pro Lys Glu Gly Lys Leu Gln Thr Glu Asn Glu Lys
        515                 520                 525
aat ata act gaa atg gcc gac gag atg gga caa gtt tct gca acc aat     1632
Asn Ile Thr Glu Met Ala Asp Glu Met Gly Gln Val Ser Ala Thr Asn
    530                 535                 540
ttg caa act atc att aac gac tat ttc ata aac cag cct cta gtg gat     1680
Leu Gln Thr Ile Ile Asn Asp Tyr Phe Ile Asn Gln Pro Leu Val Asp
545                 550                 555                 560
caa atg aca gtg ctc aaa ccg ata gga att gga aga gct tta gag caa     1728
Gln Met Thr Val Leu Lys Pro Ile Gly Ile Gly Arg Ala Leu Glu Gln
                565                 570                 575
tat tca gca ata gaa gaa ggt ggt ctt gct aca tct gcg aat cgt aac     1776
Tyr Ser Ala Ile Glu Glu Gly Gly Leu Ala Thr Ser Ala Asn Arg Asn
            580                 585                 590
ttt gat agt tgt ttg atg cat caa att gac gtt gtt cgt aaa aca ttg     1824
Phe Asp Ser Cys Leu Met His Gln Ile Asp Val Val Arg Lys Thr Leu
        595                 600                 605
aaa aag atg cct ctg cct ccg att aac gat cca tcg gat tta gac aaa     1872
Lys Lys Met Pro Leu Pro Pro Ile Asn Asp Pro Ser Asp Leu Asp Lys

    610                 615                 620

ttc cgt gat ctg atc acc aag gat tta tac gat atg aaa aaa gcc gat 1920

Phe Arg Asp Leu Ile Thr Lys Asp Leu Tyr Asp Met Lys Lys Ala Asp

625                 630                 635                 640

agc gaa aga ttc aag aat cct gtt gcc gat gtt gaa atg gaa gaa gat 1968

Ser Glu Arg Phe Lys Asn Pro Val Ala Asp Val Glu Met Glu Glu Asp

                645                 650                 655

gaa gac gat cct caa tac tat atg cct cca cag tct act tcg aga aca 2016

Glu Asp Asp Pro Gln Tyr Tyr Met Pro Pro Gln Ser Thr Ser Arg Thr

            660                 665                 670

aat tat gct agc ggc agt gaa gat gaa gtg gca aat tct gat gaa gaa 2064

Asn Tyr Ala Ser Gly Ser Glu Asp Glu Val Ala Asn Ser Asp Glu Glu

        675                 680                 685

atg gga agc agt atc tcg aga cat tcg aag caa cct acc aca cgt ggt 2112

Met Gly Ser Ser Ile Ser Arg His Ser Lys Gln Pro Thr Thr Arg Gly

    690                 695                 700

aga ggt cgt ggt gcc aga gga gca ggt gct tca cga gaa act act cgt 2160

Arg Gly Arg Gly Ala Arg Gly Ala Gly Ala Ser Arg Glu Thr Thr Arg

705                 710                 715                 720

gga aga tcc aat aaa gtt gtt tca acg cga caa atc gat tca gat gga 2208

Gly Arg Ser Asn Lys Val Val Ser Thr Arg Gln Ile Asp Ser Asp Gly

                725                 730                 735

ttc gaa att atc aat gat tcg ccg tct ccg cct cca gct tca cga tca 2256

Phe Glu Ile Ile Asn Asp Ser Pro Ser Pro Pro Pro Ala Ser Arg Ser

            740                 745                 750

acc aga gga aaa gca aga gga aaa tca gct cca tcc aag aaa agg gat 2304

Thr Arg Gly Lys Ala Arg Gly Lys Ser Ala Pro Ser Lys Lys Arg Asp

        755                 760                 765

cta agt ttc ttc taa 2319

Leu Ser Phe Phe
    770

<210> 4
<211> 772
<212> PRT
<213> Homo sapiens

<400> 4
Met Ser Ala Thr Gly Lys Val Ser Arg Pro Ile Gln Val Phe Thr Arg
1               5                   10                  15
Ala Asn Tyr Phe Phe Lys Leu Ile Phe Gln Phe Lys Val Arg Lys Tyr
            20                  25                  30
Asn Ser Leu Thr Val Glu Arg Ile Phe Ile Arg Ala Met Cys Gly Ser
        35                  40                  45
Asp Asp Ser Phe Asp Asp Phe Val Pro Asp Ser Gln Glu Pro Ala Ser
    50                  55                  60
Ser Arg Thr Arg Asn Gln Asp His Leu Asp Asp Asp Glu Val Pro Cys
65                  70                  75                  80
Ser Gln Arg Pro Asp Ala Ala Asn Asp Thr Met Val Glu Asp Leu Asp
                85                  90                  95
Glu Gly Asp Glu Pro Ala His Asp Glu Ser Glu Asp Ile Ile Lys Ile
            100                 105                 110
Leu Val Ala Thr Asp Ile His Cys Gly Tyr Gly Glu Asn Lys Ala Asn
        115                 120                 125
Ile His Met Asp Ala Val Asn Thr Phe Glu Glu Val Leu Gln Ile Ala
    130                 135                 140
Thr Glu Gln Lys Val Asp Met Ile Leu Leu Gly Gly Asp Leu Phe His
145                 150                 155                 160

Glu Asn Asn Pro Ser Arg Glu Val Gln His Arg Val Thr Gln Leu Leu
                165                 170                 175
Arg Gln Tyr Cys Leu Asn Gly Asn Pro Ile Ala Leu Glu Phe Leu Ser
            180                 185                 190
Asp Ala Ser Val Asn Phe Asn Gln Ser Val Phe Gly His Val Asn Tyr
        195                 200                 205
Tyr Asp Gln Asn Leu Asn Val Gly Leu Pro Ile Phe Thr Ile His Gly
    210                 215                 220
Asn His Asp Asp Leu Ser Gly Lys Gly Leu Thr Ala Leu Asp Leu Leu
225                 230                 235                 240
His Glu Ser Gly Leu Val Asn Leu Phe Gly Lys His Ser Asn Ile Gln
                245                 250                 255
Glu Phe Ile Val Ser Pro Ile Leu Leu Arg Lys Gly Glu Thr Arg Leu
            260                 265                 270
Ala Leu Tyr Gly Ile Gly Ser Gln Arg Asp Asp Arg Leu Val Arg Ala
        275                 280                 285
Phe Lys Asn Asn Ser Ile Ser Phe Leu Arg Pro Asn Ala Gly Ala Glu
    290                 295                 300
Asp Trp Phe Asn Leu Phe Val Leu His Gln Asn Arg Pro Arg Arg Ala
305                 310                 315                 320
Met His Arg Ser Thr Gly Asn Phe Leu Pro Glu Ser Leu Ile Pro Gln
                325                 330                 335
Phe Phe Asp Leu Leu Ile Trp Gly His Glu His Glu Cys Lys Pro Asp
            340                 345                 350
Pro Gln Tyr Val Ala Ser Ser Glu Ala Val Gly Asp Gly Phe Tyr Ile
        355                 360                 365
Leu Gln Pro Gly Ser Thr Val Ala Thr Ser Leu Thr Pro Glu Glu Ala
    370                 375                 380
Leu Gln Lys Asn Ala Phe Leu Ile Lys Ile Lys Gly Arg Lys Phe Ala

385                 390                 395                 400
Ser Lys Pro Ile Pro Leu Gln Thr Val Arg Pro Met Val Cys Asp Glu
                405                 410                 415
Leu Leu Leu Asp Lys Ile Pro Pro Gly Ser Arg Pro Ile Leu Lys Thr
            420                 425                 430
Asp Arg Pro Lys His Thr Asp Gly Arg Tyr Ile Asp Glu Ile Ala Ile
        435                 440                 445
Glu Ala Lys Ile Asn Glu Met Ile Thr Thr Ala Lys Ala Lys Arg Arg
    450                 455                 460
Pro Arg Gln Pro Glu Leu Pro Leu Ile Arg Leu Lys Val Ile Tyr Asp
465                 470                 475                 480
Gly Asp Trp Leu Asn Ile Thr Pro Ala Asn Ala Lys Arg Ile Gly Leu
                485                 490                 495
Arg Tyr Glu Asn Val Val Ala Asn Ala Val Asp Met Val Phe Ile Lys
            500                 505                 510
Lys Asn Asn Lys Pro Lys Glu Gly Lys Leu Gln Thr Glu Asn Glu Lys
        515                 520                 525
Asn Ile Thr Glu Met Ala Asp Glu Met Gly Gln Val Ser Ala Thr Asn
    530                 535                 540
Leu Gln Thr Ile Ile Asn Asp Tyr Phe Ile Asn Gln Pro Leu Val Asp
545                 550                 555                 560
Gln Met Thr Val Leu Lys Pro Ile Gly Ile Gly Arg Ala Leu Glu Gln
                565                 570                 575
Tyr Ser Ala Ile Glu Glu Gly Gly Leu Ala Thr Ser Ala Asn Arg Asn
            580                 585                 590
Phe Asp Ser Cys Leu Met His Gln Ile Asp Val Val Arg Lys Thr Leu
        595                 600                 605
Lys Lys Met Pro Leu Pro Pro Ile Asn Asp Pro Ser Asp Leu Asp Lys
    610                 615                 620

Phe Arg Asp Leu Ile Thr Lys Asp Leu Tyr Asp Met Lys Lys Ala Asp
625                 630                 635                 640
Ser Glu Arg Phe Lys Asn Pro Val Ala Asp Val Glu Met Glu Glu Asp
                645                 650                 655
Glu Asp Asp Pro Gln Tyr Tyr Met Pro Pro Gln Ser Thr Ser Arg Thr
            660                 665                 670
Asn Tyr Ala Ser Gly Ser Glu Asp Glu Val Ala Asn Ser Asp Glu Glu
        675                 680                 685
Met Gly Ser Ser Ile Ser Arg His Ser Lys Gln Pro Thr Thr Arg Gly
    690                 695                 700
Arg Gly Arg Gly Ala Arg Gly Ala Gly Ala Ser Arg Glu Thr Thr Arg
705                 710                 715                 720
Gly Arg Ser Asn Lys Val Val Ser Thr Arg Gln Ile Asp Ser Asp Gly
                725                 730                 735
Phe Glu Ile Ile Asn Asp Ser Pro Ser Pro Pro Pro Ala Ser Arg Ser
            740                 745                 750
Thr Arg Gly Lys Ala Arg Gly Lys Ser Ala Pro Ser Lys Lys Arg Asp
        755                 760                 765
Leu Ser Phe Phe
    770

<210> 5
<211> 2079
<212> DNA
<213> Saccharomyces cerevisiae

<220>
<221> CDS
<222> (1)..(2076)

<400> 5

atg gac tat cct gat cca gac aca ata agg att tta att act aca gct     48

Met Asp Tyr Pro Asp Pro Asp Thr Ile Arg Ile Leu Ile Thr Thr Ala

1               5                   10                  15

aat cat gtg ggt tac aac gaa aat gat ccc att act ggc gat gat tct     96

Asn His Val Gly Tyr Asn Glu Asn Asp Pro Ile Thr Gly Asp Asp Ser

            20                  25                  30

tgg aaa act ttc cat gaa gtc atg atg ctg gcc aaa aat aac aac gta     144

Trp Lys Thr Phe His Glu Val Met Met Leu Ala Lys Asn Asn Asn Val

        35                  40                  45

gac atg gtt gta cag tcc ggt gat ctt ttt cac gtg aat aag cct tcc     192

Asp Met Val Val Gln Ser Gly Asp Leu Phe His Val Asn Lys Pro Ser

    50                  55                  60

aag aag tca ctc tac caa gta ctg aaa act ttg aga tta tgt tgc atg     240

Lys Lys Ser Leu Tyr Gln Val Leu Lys Thr Leu Arg Leu Cys Cys Met

65                  70                  75                  80

ggt gac aag cct tgc gag tta gaa tta ttg agc gat ccc tca caa gtt     288

Gly Asp Lys Pro Cys Glu Leu Glu Leu Leu Ser Asp Pro Ser Gln Val

                85                  90                  95

ttt cac tac gat gaa ttt acc aac gtt aac tat gag gac ccc aac ttt     336

Phe His Tyr Asp Glu Phe Thr Asn Val Asn Tyr Glu Asp Pro Asn Phe

            100                 105                 110

aat att tct att ccc gta ttc ggc ata tca ggt aat cat gat gat gcg     384

Asn Ile Ser Ile Pro Val Phe Gly Ile Ser Gly Asn His Asp Asp Ala

        115                 120                 125

tcg ggg gac tca ctg ttg tgt cct atg gat ata ctt cat gcg act ggt     432

Ser Gly Asp Ser Leu Leu Cys Pro Met Asp Ile Leu His Ala Thr Gly

    130                 135                 140

cta ata aat cat ttc ggg aaa gtc atc gaa tct gat aaa ata aaa gtc 480
Leu Ile Asn His Phe Gly Lys Val Ile Glu Ser Asp Lys Ile Lys Val
145                 150                 155                 160
gtg cca tta tta ttt cag aaa ggg tcc act aag tta gca ttg tac gga 528
Val Pro Leu Leu Phe Gln Lys Gly Ser Thr Lys Leu Ala Leu Tyr Gly
                165                 170                 175
tta gcc gct gtt cgt gat gaa agg tta ttt aga act ttt aag gat ggt 576
Leu Ala Ala Val Arg Asp Glu Arg Leu Phe Arg Thr Phe Lys Asp Gly
            180                 185                 190
ggt gtc act ttt gaa gta ccg act atg cga gaa ggt gaa tgg ttt aat 624
Gly Val Thr Phe Glu Val Pro Thr Met Arg Glu Gly Glu Trp Phe Asn
        195                 200                 205
tta atg tgc gtc cat caa aat cat aca ggt cac acg aat act gca ttt 672
Leu Met Cys Val His Gln Asn His Thr Gly His Thr Asn Thr Ala Phe
    210                 215                 220
tta cct gaa cag ttc ttg cca gat ttc ctg gat atg gtg ata tgg ggt 720
Leu Pro Glu Gln Phe Leu Pro Asp Phe Leu Asp Met Val Ile Trp Gly
225                 230                 235                 240
cat gaa cat gag tgt att ccg aat ctc gta cac aat cca att aaa aat 768
His Glu His Glu Cys Ile Pro Asn Leu Val His Asn Pro Ile Lys Asn
                245                 250                 255
ttt gat gta tta caa cct ggt tca tct gta gct act tca ctt tgt gag 816
Phe Asp Val Leu Gln Pro Gly Ser Ser Val Ala Thr Ser Leu Cys Glu
            260                 265                 270
gct gag gca caa ccc aag tat gtc ttc atc ctt gac ata aag tat gga 864
Ala Glu Ala Gln Pro Lys Tyr Val Phe Ile Leu Asp Ile Lys Tyr Gly
        275                 280                 285
gaa gca cca aaa atg aca cct att cct ctt gag act ata cgg aca ttc 912
Glu Ala Pro Lys Met Thr Pro Ile Pro Leu Glu Thr Ile Arg Thr Phe

    290                 295                 300

aaa atg aaa tcc att tcg tta caa gat gtt ccc cat ttg agg cct cac 960

Lys Met Lys Ser Ile Ser Leu Gln Asp Val Pro His Leu Arg Pro His

305                 310                 315                 320

gat aaa gat gct acg tct aag tat ctt att gaa caa gtt gaa gaa atg 1008

Asp Lys Asp Ala Thr Ser Lys Tyr Leu Ile Glu Gln Val Glu Glu Met

                325                 330                 335

atc cgc gac gct aat gag gaa act aaa caa aaa tta gcg gac gat ggt 1056

Ile Arg Asp Ala Asn Glu Glu Thr Lys Gln Lys Leu Ala Asp Asp Gly

            340                 345                 350

gaa ggt gac atg gtt gcg gaa tta ccg aaa cca ttg atc aga tta cgt 1104

Glu Gly Asp Met Val Ala Glu Leu Pro Lys Pro Leu Ile Arg Leu Arg

        355                 360                 365

gtt gat tat agt gca ccc tcc aat aca caa tcc tca ata gat tac caa 1152

Val Asp Tyr Ser Ala Pro Ser Asn Thr Gln Ser Ser Ile Asp Tyr Gln

    370                 375                 380

gta gaa aac ccg cgt aga ttt agc aat cga ttt gtg gga cgt gtt gct 1200

Val Glu Asn Pro Arg Arg Phe Ser Asn Arg Phe Val Gly Arg Val Ala

385                 390                 395                 400

aac ggt aat aac gtt gtg cag ttt tat aaa aaa agg tca cct gta act 1248

Asn Gly Asn Asn Val Val Gln Phe Tyr Lys Lys Arg Ser Pro Val Thr

                405                 410                 415

aga tca aaa aaa tcc ggt ata aat gga aca agc atc agt gat aga gat 1296

Arg Ser Lys Lys Ser Gly Ile Asn Gly Thr Ser Ile Ser Asp Arg Asp

            420                 425                 430

gtt gag aaa ctt ttc agc gaa agt ggc ggt gaa cta gaa gtt caa act 1344

Val Glu Lys Leu Phe Ser Glu Ser Gly Gly Glu Leu Glu Val Gln Thr

        435                 440                 445

ttg gtt aat gat ctc ttg aac aaa atg caa cta tct tta tta cca gaa 1392

Leu Val Asn Asp Leu Leu Asn Lys Met Gln Leu Ser Leu Leu Pro Glu
    450                 455                 460
gtt ggt ttg aat gaa gca gta aag aag ttt gta gat aaa gat gag aaa 1440
Val Gly Leu Asn Glu Ala Val Lys Lys Phe Val Asp Lys Asp Glu Lys
465                 470                 475                 480
aca gct ctt aaa gaa ttt att agc cat gaa ata tcg aac gaa gtt gga 1488
Thr Ala Leu Lys Glu Phe Ile Ser His Glu Ile Ser Asn Glu Val Gly
                485                 490                 495
ata tta tct acg aat gaa gaa ttt ctg aga aca gat gat gca gag gaa 1536
Ile Leu Ser Thr Asn Glu Glu Phe Leu Arg Thr Asp Asp Ala Glu Glu
            500                 505                 510
atg aaa gcg ctt ata aaa cag gtt aag cgt gct aac agt gtt agg ccg 1584
Met Lys Ala Leu Ile Lys Gln Val Lys Arg Ala Asn Ser Val Arg Pro
        515                 520                 525
act ccc cct aaa gaa aat gat gag aca aat ttc gca ttc aat ggt aat 1632
Thr Pro Pro Lys Glu Asn Asp Glu Thr Asn Phe Ala Phe Asn Gly Asn
    530                 535                 540
ggg cta gat tcc ttc cgg tct agt aat aga gaa gta aga act gga tct 1680
Gly Leu Asp Ser Phe Arg Ser Ser Asn Arg Glu Val Arg Thr Gly Ser
545                 550                 555                 560
cca gac att acc caa tca cat gtt gat aat gaa tca aga ata acc cat 1728
Pro Asp Ile Thr Gln Ser His Val Asp Asn Glu Ser Arg Ile Thr His
                565                 570                 575
att agt caa gcg gaa agc agt aag cca acg agc aaa ccc aaa cga gtg 1776
Ile Ser Gln Ala Glu Ser Ser Lys Pro Thr Ser Lys Pro Lys Arg Val
            580                 585                 590
cga act gca acg aaa aag aaa att cct gct ttt tca gac tca act gtc 1824
Arg Thr Ala Thr Lys Lys Lys Ile Pro Ala Phe Ser Asp Ser Thr Val
        595                 600                 605

ata tcc gat gca gaa aat gaa ctc ggt gat aat aac gat gct caa gat 1872
Ile Ser Asp Ala Glu Asn Glu Leu Gly Asp Asn Asn Asp Ala Gln Asp
    610                 615                 620
gat gtt gat att gat gag aat gac ata att atg gtc agt act gac gaa 1920
Asp Val Asp Ile Asp Glu Asn Asp Ile Ile Met Val Ser Thr Asp Glu
625                 630                 635                 640
gag gac gct agt tat ggt tta ctt aat ggt cga aaa aca aaa aca aag 1968
Glu Asp Ala Ser Tyr Gly Leu Leu Asn Gly Arg Lys Thr Lys Thr Lys
                645                 650                 655
act cgt tct gct gcg agc acc aaa acc gct tcc aga agg gga aaa gga 2016
Thr Arg Ser Ala Ala Ser Thr Lys Thr Ala Ser Arg Arg Gly Lys Gly
            660                 665                 670
aga gca tca agg acg cca aag acg gat att ctt gga agt ctc ctt gct 2064
Arg Ala Ser Arg Thr Pro Lys Thr Asp Ile Leu Gly Ser Leu Leu Ala
        675                 680                 685
aag aaa aga aaa tag 2079
Lys Lys Arg Lys
    690

<210> 6
<211> 692
<212> PRT
<213> Saccharomyces cerevisiae

<400> 6
Met Asp Tyr Pro Asp Pro Asp Thr Ile Arg Ile Leu Ile Thr Thr Ala
1               5                   10                  15
Asn His Val Gly Tyr Asn Glu Asn Asp Pro Ile Thr Gly Asp Asp Ser
            20                  25                  30

Trp Lys Thr Phe His Glu Val Met Met Leu Ala Lys Asn Asn Asn Val
        35                  40                  45
Asp Met Val Val Gln Ser Gly Asp Leu Phe His Val Asn Lys Pro Ser
    50                  55                  60
Lys Lys Ser Leu Tyr Gln Val Leu Lys Thr Leu Arg Leu Cys Cys Met
65                  70                  75                  80
Gly Asp Lys Pro Cys Glu Leu Glu Leu Leu Ser Asp Pro Ser Gln Val
                85                  90                  95
Phe His Tyr Asp Glu Phe Thr Asn Val Asn Tyr Glu Asp Pro Asn Phe
            100                 105                 110
Asn Ile Ser Ile Pro Val Phe Gly Ile Ser Gly Asn His Asp Asp Ala
        115                 120                 125
Ser Gly Asp Ser Leu Leu Cys Pro Met Asp Ile Leu His Ala Thr Gly
    130                 135                 140
Leu Ile Asn His Phe Gly Lys Val Ile Glu Ser Asp Lys Ile Lys Val
145                 150                 155                 160
Val Pro Leu Leu Phe Gln Lys Gly Ser Thr Lys Leu Ala Leu Tyr Gly
                165                 170                 175
Leu Ala Ala Val Arg Asp Glu Arg Leu Phe Arg Thr Phe Lys Asp Gly
            180                 185                 190
Gly Val Thr Phe Glu Val Pro Thr Met Arg Glu Gly Glu Trp Phe Asn
        195                 200                 205
Leu Met Cys Val His Gln Asn His Thr Gly His Thr Asn Thr Ala Phe
    210                 215                 220
Leu Pro Glu Gln Phe Leu Pro Asp Phe Leu Asp Met Val Ile Trp Gly
225                 230                 235                 240
His Glu His Glu Cys Ile Pro Asn Leu Val His Asn Pro Ile Lys Asn
                245                 250                 255
Phe Asp Val Leu Gln Pro Gly Ser Ser Val Ala Thr Ser Leu Cys Glu

            260                 265                 270
Ala Glu Ala Gln Pro Lys Tyr Val Phe Ile Leu Asp Ile Lys Tyr Gly
        275                 280                 285
Glu Ala Pro Lys Met Thr Pro Ile Pro Leu Glu Thr Ile Arg Thr Phe
    290                 295                 300
Lys Met Lys Ser Ile Ser Leu Gln Asp Val Pro His Leu Arg Pro His
305. 310 315 320
Asp Lys Asp Ala Thr Ser Lys Tyr Leu Ile Glu Gln Val Glu Glu Met
325 330 335
Ile Arg Asp Ala Asn Glu Glu Thr Lys Gln Lys Leu Ala Asp Asp Gly
340 345 350
Glu Gly Asp Met Val Ala Glu Leu Pro Lys Pro Leu Ile Arg Leu Arg
355 360 365
Val Asp Tyr Ser Ala Pro Ser Asn Thr Gln Ser Ser Ile Asp Tyr Gln
370 375 380
Val Glu Asn Pro Arg Arg Phe Ser Asn Arg Phe Val Gly Arg Val Ala
385 390 395 400
Asn Gly Asn Asn Val Val Gln Phe Tyr Lys Lys Arg Ser Pro Val Thr
405 410 415
Arg Ser Lys Lys Ser Gly Ile Asn Gly Thr Ser Ile Ser Asp Arg Asp
420 425 430
Val Glu Lys Leu Phe Ser Glu Ser Gly Gly Glu Leu Glu Val Gln Thr
435 440 445
Leu Val Asn Asp Leu Leu Asn Lys Met Gln Leu Ser Leu Leu Pro Glu
450 455 460
Val Gly Leu Asn Glu Ala Val Lys Lys Phe Val Asp Lys Asp Glu Lys
465 470 475 480
Thr Ala Leu Lys Glu Phe Ile Ser His Glu Ile Ser Asn Glu Val Gly
485 490 495

Ile Leu Ser Thr Asn Glu Glu Phe Leu Arg Thr Asp Asp Ala Glu Glu
500 505 510
Met Lys Ala Leu Ile Lys Gln Val Lys Arg Ala Asn Ser Val Arg Pro
515 520 525
Thr Pro Pro Lys Glu Asn Asp Glu Thr Asn Phe Ala Phe Asn Gly Asn
530 535 540
Gly Leu Asp Ser Phe Arg Ser Ser Asn Arg Glu Val Arg Thr Gly Ser
545 550 555 560
Pro Asp Ile Thr Gln Ser His Val Asp Asn Glu Ser Arg Ile Thr His
565 570 575
Ile Ser Gln Ala Glu Ser Ser Lys Pro Thr Ser Lys Pro Lys Arg Val
580 585 590
Arg Thr Ala Thr Lys Lys Lys Ile Pro Ala Phe Ser Asp Ser Thr Val
595 600 605
Ile Ser Asp Ala Glu Asn Glu Leu Gly Asp Asn Asn Asp Ala Gln Asp
610 615 620
Asp Val Asp Ile Asp Glu Asn Asp Ile Ile Met Val Ser Thr Asp Glu
625 630 635 640
Glu Asp Ala Ser Tyr Gly Leu Leu Asn Gly Arg Lys Thr Lys Thr Lys
645 650 655
Thr Arg Ser Ala Ala Ser Thr Lys Thr Ala Ser Arg Arg Gly Lys Gly
660 665 670
Arg Ala Ser Arg Thr Pro Lys Thr Asp Ile Leu Gly Ser Leu Leu Ala
675 680 685
Lys Lys Arg Lys
690

<210> 7
<211> 1932

<212> DNA

<213> Saccharomyces cerevisiae

<220>

<221> CDS

<222> (1)..(1929)

<400> 7

atg gac tat cct gat cca gac aca ata agg att tta att act aca gat 48

Met Asp Tyr Pro Asp Pro Asp Thr Ile Arg Ile Leu Ile Thr Thr Asp

1               5                   10                  15

aat cat gtg ggt tac aac gaa aat gat ccc att act ggc gat gat tct 96

Asn His Val Gly Tyr Asn Glu Asn Asp Pro Ile Thr Gly Asp Asp Ser

            20                  25                  30

tgg aaa act ttc cat gaa gtc atg atg ctg gcc aaa aat aac aac gta 144

Trp Lys Thr Phe His Glu Val Met Met Leu Ala Lys Asn Asn Asn Val

        35                  40                  45

gac atg gtt gta cag tcc ggt gat ctt ttt cac gtg aat aag cct tcc 192

Asp Met Val Val Gln Ser Gly Asp Leu Phe His Val Asn Lys Pro Ser

    50                  55                  60

aag aag tca ctc tac caa gta ctg aaa act ttg aga tta tgt tgc atg 240

Lys Lys Ser Leu Tyr Gln Val Leu Lys Thr Leu Arg Leu Cys Cys Met

65                  70                  75                  80

ggt gac aag cct tgc gag tta gaa tta ttg agc gat ccc tca caa gtt 288

Gly Asp Lys Pro Cys Glu Leu Glu Leu Leu Ser Asp Pro Ser Gln Val

                85                  90                  95

ttt cac tac gat gaa ttt acc aac gtt aac tat gag gac ccc aac ttt 336

Phe His Tyr Asp Glu Phe Thr Asn Val Asn Tyr Glu Asp Pro Asn Phe

            100                 105                 110

aat att tct att ccc gta ttc ggc ata tca ggt aat cat gat gat gcg 384
Asn Ile Ser Ile Pro Val Phe Gly Ile Ser Gly Asn His Asp Asp Ala
        115                 120                 125
tcg ggg gac tca ctg ttg tgt cct atg gat ata ctt cat gcg act ggt 432
Ser Gly Asp Ser Leu Leu Cys Pro Met Asp Ile Leu His Ala Thr Gly
    130                 135                 140
cta ata aat cat ttc ggg aaa gtc atc gaa tct gat aaa ata aaa gtc 480
Leu Ile Asn His Phe Gly Lys Val Ile Glu Ser Asp Lys Ile Lys Val
145                 150                 155                 160
gtg cca tta tta ttt cag aaa ggg tcc act aag tta gca ttg tac gga 528
Val Pro Leu Leu Phe Gln Lys Gly Ser Thr Lys Leu Ala Leu Tyr Gly
                165                 170                 175
tta gcc gct gtt cgt gat gaa agg tta ttt aga act ttt aag gat ggt 576
Leu Ala Ala Val Arg Asp Glu Arg Leu Phe Arg Thr Phe Lys Asp Gly
            180                 185                 190
ggt gtc act ttt gaa gta ccg act atg cga gaa ggt gaa tgg ttt aat 624
Gly Val Thr Phe Glu Val Pro Thr Met Arg Glu Gly Glu Trp Phe Asn
        195                 200                 205
tta atg tgc gtc cat caa aat cat aca ggt cac acg aat act gca ttt 672
Leu Met Cys Val His Gln Asn His Thr Gly His Thr Asn Thr Ala Phe
    210                 215                 220
tta cct gaa cag ttc ttg cca gat ttc ctg gat atg gtg ata tgg ggt 720
Leu Pro Glu Gln Phe Leu Pro Asp Phe Leu Asp Met Val Ile Trp Gly
225                 230                 235                 240
cat gaa cat gag tgt att ccg aat ctc gta cac aat cca att aaa aat 768
His Glu His Glu Cys Ile Pro Asn Leu Val His Asn Pro Ile Lys Asn
                245                 250                 255
ttt gat gta tta caa cct ggt tca tct gta gct act tca ctt tgt gag 816
Phe Asp Val Leu Gln Pro Gly Ser Ser Val Ala Thr Ser Leu Cys Glu

            260                 265                 270
gct gag gca caa ccc aag tat gtc ttc atc ctt gac ata aag tat gga 864
Ala Glu Ala Gln Pro Lys Tyr Val Phe Ile Leu Asp Ile Lys Tyr Gly
        275                 280                 285
gaa gca cca aaa atg aca cct att cct ctt gag act ata cgg aca ttc 912
Glu Ala Pro Lys Met Thr Pro Ile Pro Leu Glu Thr Ile Arg Thr Phe
    290                 295                 300
aaa atg aaa tcc att tcg tta caa gat gtt ccc cat ttg agg cct cac 960
Lys Met Lys Ser Ile Ser Leu Gln Asp Val Pro His Leu Arg Pro His
305                 310                 315                 320
gat aaa gat gct acg tct aag tat ctt att gaa caa gtt gaa gaa atg 1008
Asp Lys Asp Ala Thr Ser Lys Tyr Leu Ile Glu Gln Val Glu Glu Met
                325                 330                 335
atc cgc gac gct aat gag gaa act aaa caa aaa tta gcg gac gat ggt 1056
Ile Arg Asp Ala Asn Glu Glu Thr Lys Gln Lys Leu Ala Asp Asp Gly
            340                 345                 350
gaa ggt gac atg gtt gcg gaa tta ccg aaa cca ttg atc aga tta cgt 1104
Glu Gly Asp Met Val Ala Glu Leu Pro Lys Pro Leu Ile Arg Leu Arg
        355                 360                 365
gtt gat tat agt gca ccc tcc aat aca caa tcc tca ata gat tac caa 1152
Val Asp Tyr Ser Ala Pro Ser Asn Thr Gln Ser Ser Ile Asp Tyr Gln
    370                 375                 380
gta gaa aac ccg cgt aga ttt agc aat cga ttt gtg gga cgt gtt gct 1200
Val Glu Asn Pro Arg Arg Phe Ser Asn Arg Phe Val Gly Arg Val Ala
385                 390                 395                 400
aac ggt aat aac gtt gtg cag ttt tat aaa aaa agg tca cct gta act 1248
Asn Gly Asn Asn Val Val Gln Phe Tyr Lys Lys Arg Ser Pro Val Thr
                405                 410                 415
aga tca aaa aaa tcc ggt ata aat gga aca agc atc agt gat aga gat 1296

Arg Ser Lys Lys Ser Gly Ile Asn Gly Thr Ser Ile Ser Asp Arg Asp
            420                 425                 430
gtt gag aaa ctt ttc agc gaa agt ggc ggt gaa cta gaa gtt caa act 1344
Val Glu Lys Leu Phe Ser Glu Ser Gly Gly Glu Leu Glu Val Gln Thr
        435                 440                 445
ttg gtt aat gat ctc ttg aac aaa atg caa cta tct tta tta cca gaa 1392
Leu Val Asn Asp Leu Leu Asn Lys Met Gln Leu Ser Leu Leu Pro Glu
    450                 455                 460
gtt ggt ttg aat gaa gca gta aag aag ttt gta gat aaa gat gag aaa 1440
Val Gly Leu Asn Glu Ala Val Lys Lys Phe Val Asp Lys Asp Glu Lys
465                 470                 475                 480
aca gct ctt aaa gaa ttt att agc cat gaa ata tcg aac gaa gtt gga 1488
Thr Ala Leu Lys Glu Phe Ile Ser His Glu Ile Ser Asn Glu Val Gly
                485                 490                 495
ata tta tct acg aat gaa gaa ttt ctg aga aca gat gat gca gag gaa 1536
Ile Leu Ser Thr Asn Glu Glu Phe Leu Arg Thr Asp Asp Ala Glu Glu
            500                 505                 510
atg aaa gcg ctt ata aaa cag gtt aag cgt gct aac agt gtt agg ccg 1584
Met Lys Ala Leu Ile Lys Gln Val Lys Arg Ala Asn Ser Val Arg Pro
        515                 520                 525
act ccc cct aaa gaa aat gat gag aca aat ttc gca ttc aat ggt aat 1632
Thr Pro Pro Lys Glu Asn Asp Glu Thr Asn Phe Ala Phe Asn Gly Asn
    530                 535                 540
ggg cta gat tcc ttc cgg tct agt aat aga gaa gta aga act gga tct 1680
Gly Leu Asp Ser Phe Arg Ser Ser Asn Arg Glu Val Arg Thr Gly Ser
545                 550                 555                 560
cca gac att acc caa tca cat gtt gat aat gaa tca aga ata acc cat 1728
Pro Asp Ile Thr Gln Ser His Val Asp Asn Glu Ser Arg Ile Thr His
                565                 570                 575

att agt caa gcg gaa agc agt aag cca acg agc aaa ccc aaa cga gtg 1776
Ile Ser Gln Ala Glu Ser Ser Lys Pro Thr Ser Lys Pro Lys Arg Val
            580                 585                 590
cga act gca acg aaa aag aaa att cct gct ttt tca gac tca act gtc 1824
Arg Thr Ala Thr Lys Lys Lys Ile Pro Ala Phe Ser Asp Ser Thr Val
        595                 600                 605
ata tcc gat gca gaa aat gaa ctc ggt gat aat aac gat gct caa gat 1872
Ile Ser Asp Ala Glu Asn Glu Leu Gly Asp Asn Asn Asp Ala Gln Asp
    610                 615                 620
gat gtt gat att gat gag aat gac ata att atg gtc agt act gac gaa 1920
Asp Val Asp Ile Asp Glu Asn Asp Ile Ile Met Val Ser Thr Asp Glu
625                 630                 635                 640
gag gac gcc tag 193[?]
Glu Asp Ala

<210> 8
<211> 643
<212> PRT
<213> Saccharomyces cerevisiae

<400> 8
Met Asp Tyr Pro Asp Pro Asp Thr Ile Arg Ile Leu Ile Thr Thr Asp
1               5                   10                  15
Asn His Val Gly Tyr Asn Glu Asn Asp Pro Ile Thr Gly Asp Asp Ser
            20                  25                  30
Trp Lys Thr Phe His Glu Val Met Met Leu Ala Lys Asn Asn Asn Val
        35                  40                  45
Asp Met Val Val Gln Ser Gly Asp Leu Phe His Val Asn Lys Pro Ser
    50                  55                  60

Lys Lys Ser Leu Tyr Gln Val Leu Lys Thr Leu Arg Leu Cys Cys Met
65                  70                  75                  80
Gly Asp Lys Pro Cys Glu Leu Glu Leu Leu Ser Asp Pro Ser Gln Val
                85                  90                  95
Phe His Tyr Asp Glu Phe Thr Asn Val Asn Tyr Glu Asp Pro Asn Phe
            100                 105                 110
Asn Ile Ser Ile Pro Val Phe Gly Ile Ser Gly Asn His Asp Asp Ala
        115                 120                 125
Ser Gly Asp Ser Leu Leu Cys Pro Met Asp Ile Leu His Ala Thr Gly
    130                 135                 140
Leu Ile Asn His Phe Gly Lys Val Ile Glu Ser Asp Lys Ile Lys Val
145                 150                 155                 160
Val Pro Leu Leu Phe Gln Lys Gly Ser Thr Lys Leu Ala Leu Tyr Gly
                165                 170                 175
Leu Ala Ala Val Arg Asp Glu Arg Leu Phe Arg Thr Phe Lys Asp Gly
            180                 185                 190
Gly Val Thr Phe Glu Val Pro Thr Met Arg Glu Gly Glu Trp Phe Asn
        195                 200                 205
Leu Met Cys Val His Gln Asn His Thr Gly His Thr Asn Thr Ala Phe
    210                 215                 220
Leu Pro Glu Gln Phe Leu Pro Asp Phe Leu Asp Met Val Ile Trp Gly
225                 230                 235                 240
His Glu His Glu Cys Ile Pro Asn Leu Val His Asn Pro Ile Lys Asn
                245                 250                 255
Phe Asp Val Leu Gln Pro Gly Ser Ser Val Ala Thr Ser Leu Cys Glu
            260                 265                 270
Ala Glu Ala Gln Pro Lys Tyr Val Phe Ile Leu Asp Ile Lys Tyr Gly
        275                 280                 285
Glu Ala Pro Lys Met Thr Pro Ile Pro Leu Glu Thr Ile Arg Thr Phe

    290                 295                 300
Lys Met Lys Ser Ile Ser Leu Gln Asp Val Pro His Leu Arg Pro His
305                 310                 315                 320
Asp Lys Asp Ala Thr Ser Lys Tyr Leu Ile Glu Gln Val Glu Glu Met
                325                 330                 335
Ile Arg Asp Ala Asn Glu Glu Thr Lys Gln Lys Leu Ala Asp Asp Gly
            340                 345                 350
Glu Gly Asp Met Val Ala Glu Leu Pro Lys Pro Leu Ile Arg Leu Arg
        355                 360                 365
Val Asp Tyr Ser Ala Pro Ser Asn Thr Gln Ser Ser Ile Asp Tyr Gln
    370                 375                 380
Val Glu Asn Pro Arg Arg Phe Ser Asn Arg Phe Val Gly Arg Val Ala
385                 390                 395                 400
Asn Gly Asn Asn Val Val Gln Phe Tyr Lys Lys Arg Ser Pro Val Thr
                405                 410                 415
Arg Ser Lys Lys Ser Gly Ile Asn Gly Thr Ser Ile Ser Asp Arg Asp
            420                 425                 430
Val Glu Lys Leu Phe Ser Glu Ser Gly Gly Glu Leu Glu Val Gln Thr
        435                 440                 445
Leu Val Asn Asp Leu Leu Asn Lys Met Gln Leu Ser Leu Leu Pro Glu
    450                 455                 460
Val Gly Leu Asn Glu Ala Val Lys Lys Phe Val Asp Lys Asp Glu Lys
465                 470                 475                 480
Thr Ala Leu Lys Glu Phe Ile Ser His Glu Ile Ser Asn Glu Val Gly
                485                 490                 495
Ile Leu Ser Thr Asn Glu Glu Phe Leu Arg Thr Asp Asp Ala Glu Glu
            500                 505                 510
Met Lys Ala Leu Ile Lys Gln Val Lys Arg Ala Asn Ser Val Arg Pro
        515                 520                 525

Thr Pro Pro Lys Glu Asn Asp Glu Thr Asn Phe Ala Phe Asn Gly Asn
    530                 535                 540
Gly Leu Asp Ser Phe Arg Ser Ser Asn Arg Glu Val Arg Thr Gly Ser
545                 550                 555                 560
Pro Asp Ile Thr Gln Ser His Val Asp Asn Glu Ser Arg Ile Thr His
                565                 570                 575
Ile Ser Gln Ala Glu Ser Ser Lys Pro Thr Ser Lys Pro Lys Arg Val
            580                 585                 590
Arg Thr Ala Thr Lys Lys Lys Ile Pro Ala Phe Ser Asp Ser Thr Val
        595                 600                 605
Ile Ser Asp Ala Glu Asn Glu Leu Gly Asp Asn Asn Asp Ala Gln Asp
    610                 615                 620
Asp Val Asp Ile Asp Glu Asn Asp Ile Ile Met Val Ser Thr Asp Glu
625                 630                 635                 640
Glu Asp Ala

<210> 9
<211> 20
<212> DNA
<213> Artificial Sequence

<220>
<223> Description of Artificial Sequence:synthetic DNA

<400> 9
gtgccattat tatttcagaa 20

<210> 10
<211> 20

<212> DNA
<213> Artificial Sequence

<220>
<223> Description of Artificial Sequence:synthetic DNA

<400> 10
ctggtaataa agatagttgc 20

<210> 11
<211> 20
<212> DNA
<213> Artificial Sequence

<220>
<223> Description of Artificial Sequence:synthetic DNA

<400> 11
atggactatc ctgatccaga 20

<210> 12
<211> 20
<212> DNA
<213> Artificial Sequence

<220>
<223> Description of Artificial Sequence:synthetic DNA

<400> 12

gggatcaagt acaactattt tc 22

<210> 13
<211> 20
<212> DNA
<213> Artificial Sequence

<220>
<223> Description of Artificial Sequence:synthetic DNA

<400> 13
gtgtgtgtgt gtgtgtgtgt 20

## Description

### TECHNICAL FIELD

This invention relates to a method of controlling telomere length wherein physiological activity of endogenous Mrell protein in a eukaryotic cell is modified, a telomere length controlling agent comprising as an active component a substance which modifies physiological activity of endogenous Mrell protein in a eukaryotic cell, and a gene therapeutic agent for telomere length-associated diseases comprising as an active component a substance which modifies physiological activity of endogenous Mrell protein in a eukaryotic cell.

### BACKGROUND ART

Telomeres are functional constructs existing at the ends of double-stranded DNA constituting filamentous chromosomes in a eukaryotic cell. It is known that this construct is generally composed of simple repeats, and plays an important role in preventing chromosomal aberration due to chromosome fusion, pairing of homologous chromosomes during myosis, control of gene expression, and determination of forms of chromosomes in a nucleus.

In general, replication of a double-stranded DNA is carried out by synthesizing a RNA primer by primase and at an end thereof by elongation-synthesis of a DNA chain by DNA polymerase. The RNA primer is remeved by 5'-3' exonuclease activity of the DNA polymerase, and at the same time the removed part is replaced with the DNA chain by the DNA polymerase. However, in the case of a filamentous chromosome, there is no replacement of the RNA primer at the 5'-most end of a nascent chain with a DNA chain, and thus when replication is completed, the chromosome is shortened by the size of the RNA primer. Therefore, every repeat of cell division progressively shortens the chromosomes from its end in the daughter cells, and finally the chromosomes become unstable, resulting in death of the cell. On the other hand, it has been shown that in an immortalized cell, telomerase which can elongate telomeres is highly expressed, thus telomere lengths can not be reduced. Consequently, intensive studies are made on regulators for cell life span or anticancer agents which involve the telomerase activity in their mechanisms.

However, recently it has been shown that in yeasts and mice etc. even when their telomerase genes are disrupted, it takes time for shortening their teromeres [Blasco, M. A., et al.: Cell, 91:25-34 (1997); Rudolph, K.L., et al.: Cell, 96:701-12 (1999); Herrera, E., et al. :Embo J, 18:2950-60(1999)], and that even a variant of a gene which is not directly related to telomerase activity, results in shortening or elongation of telomeres [Boulton, S. J., et al.:Embo J, 17:1819-28 (1998); Furuse, M., et al.: Embo J, 17:6412-25(1998); Wilson, S. et al.: Nucleic Acids Res, 27:2655-61 (1999)]. Thus, it is pointed out that there are potentially mechanisms other than telomerase for maintaining telomeres [Nakamura, T.M., et al.: Science, 282:493-6 (1998); Reddel, R.R., et al. A review. Biochemistry (Mosc), 62:1254-62 (1997)]. In particular, it has been reported that a Mrell-Rad50-Xrs2 complex which is known to be engaged in repair of double-stranded DNA cleavage etc. is also associated with controlling telomere length [Boulton, S.J., et al.: Embo J, 17: 1819-28 (1998); Furuse, M., et al.: Embo J, 17:6412-25(1998); Le, S., et al.: Genetics, 152:143-52 (1999)]. A Mrell -Rad50-Xrs2 (Nbs1) complex [Ajimura, M., et al.: Genetics, 133:51-66 (1993); Johzuka, K., et al.: Genetics, 139:1521-32 (1995)] is a protein complex formed with Mrell as a core which has a strong DNA binding activity and functions as a double-stranded DNA exonuclease and a single-stranded endonuclease [Furuse, M., et al. :Embo J, 17:6412-25 (1998); Paull, T. T., et al. : Mol Cell, 1:969-79 (1998); Usui, T., et al. : Cell, 95:705-16 (1998); Trujillo, K. M., et al. :J Biol Chem, 273:21447-50 (1998)]. The complex functions as an essential enzyme to initiation reaction for repair of DNA double-stranded cleavage or chromosomeal recombination. In budding yeast, Saccharomyces cerevisiae, or fission yeast, Schizosaccharomyces pombe, when even one of the proteins constituting this enzyme-complex is absent, notable shortening of telomeres is observed [Boulton, S.J., et al.: Embo J, 17:1819-28(1998)]. Homologs of all the constituent proteins of the Mrell-Rad50-Xrs2(Nbs1) complex exist in humans [Dolganov, G. M., et al. :Mol Cell Biol, 16:4832-41 (1996); Petrini, J.H., et al.: Genomics, 29:80-6 (1995)], and it is thought that they have a common role in all eukaryotes from yeast to human.

### DISCLOSURE OF THE INVENTION

It is an object of the present invention to provide a method of controlling telomere length wherein the method comprises modifying physiological activity of endogenous Mrell protein in a eukaryotic cell.

As a result of intensive studies based on the above subject, the inventors of the present invention have found that modifying physiological activity of endogenous Mrell protein in a eukaryotic cell enables control of telomere length of the cell, thus accomplishing the present invention.

Namely, the present invention is a method of controlling telomere length wherein the method comprises modifying physiological activity of endogenous Mrell protein in a eukaryotic cell. Herein, the modification of the physiological activities of the endogenous Mrell protein is carried out by introducing into a cell a DNA encoding foreign Mrell protein, or a DNA encoding a protein wherein the nuclease domain or the C-terminal domain of the foreign Mrell protein is modified, in a state such that the DNA can be expressed.

Further, the present invention is a telomere length controlling agent or a gene therapeutic agent for telomere-associated diseases (e.g. melanoma, hepatoma, breast cancer, gastric cancer, brain tumor, and cell senescent diseases) comprising as an active agent a substance which modifies physiological activity of endogenous Mrell protein in a eukaryotic cell. Herein, the substance which modifies physiological activity of endogenous Mrell protein in the eukaryotic cell may include a DNA construct which comprises a DNA encoding foreign Mrell protein, or a DNA encoding a protein wherein a nuclease domain or a C-terminal domain of foreign Mrell protein is modified, in a state such that the DNA can be expressed.

In the present invention, the following protein (a) or (b) is provided as foreign Mrell protein:
(a) a protein comprising an amino acid sequence represented by SEQ ID NO: 2 or 4; or
(b) a protein comprising an amino acid sequence which is the amino acid sequence represented by SEQ ID NO: 2 or 4 from, in, or to which one or more amino acids are deleted, substituted or added, and having physiological activity of Mrell protein.
   Further, in the present invention, the following DNA (c) or (d) is provided as the DNA encoding foreign Mrell protein:
(c) a DNA comprising a nucleotide sequence represented by SEQ ID NO: 1 or 3; or
(d) a DNA which can hybridize with the DNA of (c) under a stringent condition and encodes a protein having physiological activity of Mrell protein.
   Furthermore, in the present invention, the following protein (e) or (f) is provided as a protein wherein a nuclease domain of the foreign Mrell protein is modified:
(e) a protein comprising an amino acid sequence represented by SEQ ID NO: 6; or
(f) a protein comprising an amino acid sequence which is the amino acid sequence represented by SEQ ID NO: 6 from, in, or to which one or more amino acids are deleted, substituted or added, and having physiological activity (except nuclease activity) of Mrell protein.
   Moreover, in the present invention, the following DNA (g) or (h) is provided as DNA encoding a protein wherein the nuclease domain of the foreign Mrell protein is modified:
(g) a DNA comprising a nucleotide sequence represented by SEQ ID No: 5; or
(h) a DNA which can hybridize with the DNA of (g) under a stringent condition and encodes a protein having physiological activity (except nuclease activity)of Mrell protein.
   Further, in the present invention, the following protein (i) or (j) is provided as a protein wherein a C-terminal domain of the foreign Mrell protein is modified:
(i) a protein comprising an amino acid sequence represented by SEQ ID NO: 8; or
(j) a protein comprising an amino acid sequence which is the amino acid sequence represented by SEQ ID NO: 8 from, in or to which one or more amino acids are deleted, substituted or added, and having physiological activity (except double-stranded DNA binding activity) of Mrell protein.
   Still further, in the present invention, the following DNA (k) or (1) is provided as DNA encoding a protein wherein the C-terminal domain of the foreign Mrell protein is modified:
(k) a DNA comprising a nucleotide sequence represented by SEQ ID NO: 7; or
(l) a DNA which can hybridize with the DNA of (k) and encodes a protein having physiological activity (except double-stranded DNA binding activity)of Mrell protein.

The present invention will hereinafter be described in detail.

A method of controlling telomere length according to the present invention is based on modification of physiological activity of endogenous Mrell protein in a eukaryotic cell. Herein, the term "controlling telomere length" means shortening , retaining or elongating telomere length in cells. The term "endogenous Mrell protein" means a protein which natively exists in a cell and has physiological activity of Mrell protein. The term "physiological activity of Mrell protein" means at least one of the following activities: the ability of forming a complex with Rad50 protein and Xrs2 (Nbs1) protein, double-stranded DNA binding activity, double-stranded DNA exonuclease activity and single-stranded DNA endonuclease activity. The double-stranded DNA exonuclease activity and the single-stranded DNA endonuclease activity both as a whole are referred to as the nuclease activity. Further, "modification" means to improve, reduce or delete, partially or wholly physiological activity of the endogenous Mrell protein. For example, shortening of telomeres can be carried out by introducing into a cell a DNA encoding a full length of foreign Mrell protein or a DNA encoding a protein wherein the nuclease domain of the foreign Mrell protein is modified, in a state such that the DNA can be expressed. Here, "a state such that the DNA can be expressed" means a state wherein the DNA is comprised in a vector together with a DNA region concerning the expression, e.g. a promoter so that a protein encoded by the DNA can be produced in the cell into which the DNA has been introduced. In contrast, the retaining or increasing of telomere length is carried out by introducing into a cell a DNA encoding a protein wherein the C-terminal domain of the foreign Mrell protein is modified, in a state such that the DNA can be expressed. Here the term "foreign Mrell protein" means Mrell protein derived from outside of a target cell, and the term "modify" means that one or more amino acids is deleted, substituted or added from, in or to a functional domain.

### 1. Preparation of a DNA encoding foreign Mrell protein

### (1) Sources of a DNA encoding foreign Mrell protein

The source of a DNA encoding foreign Mrell protein which can be used for controlling telomere length is any organism-derived cell as long as it has a DNA encoding Mrell protein, without particular limitation. Examples thereof include cells derived from various eukaryotes e.g. human (Homo sapiens), a mouse (Mus musculus), a South African clawed frog (Xenopus laevis), a fly (Drosophila melanogaster), Arabidopsis thaliana, Saccharomyces cerevisiae, Coprinus cinereus, Schizosaccharomyces pombe. Preferably a DNA encoding foreign Mrell protein to be used for controlling telomere length can be derived from the same species as that of a target cell for telomere length control. In regard to Schizosaccharomyces pombe, a protein corresponding to Mrell protein is called Rad32. For example, for controlling telomere length of human cells, it is preferable to use a DNA encoding human Mrell protein, and for controlling the telomere lengths of Saccharomyce cerevisiae cells, it is preferable to use a DNA encoding Mrell protein from Saccharomyce cerevisiae.

### (2) Preparation of a DNA encoding foreign Mrell protein

A DNA encoding foreign Mrell protein can be obtained by screening a clone including a DNA encoding Mrell protein from a genome DNA library or a cDNA library which has been prepared from a cell derived from organisms described in the section (1) above, or by direct amplification by PCR using a genome DNA or a cDNA as a template.

For example, for obtaining the DNA encoding Mrell protein by screening a cDNA library, initially mRNAs are prepared from cells of the organisms described in section (1) above, by a method such as the guanidine thiocyanate method. Next, a synthetic DNA primer including an oligo-dT sequence is hybridized, then a single-stranded cDNA is synthesized by a reverse transcriptase. Thereafter, using E coli DNA polymerase I, E coli DNA ligase and RNaseH, double-stranded cDNAs are synthesized by a conventional method. Next, after blunting cDNA ends by T4DNA polymerase, a DNA adaptor e.g. EcoRI adaptor, is added to both ends of the cDNA chains by T4DNA ligase. Then, the cDNA chains added by the DNA adaptors are inserted into a restriction site of a commercially available λ phage vector (e.g. λZAP available from Stratagene) or a plasmid vector (e.g. pGEM2 available from PromegaBiotech) in accordance with a conventional method. Thus, a group of recombinant λ phage DNAs or recombinant plasmid DNAs can be obtained.

Subsequently, in regard to the group of therecombinant λ phage DNAs, in vitro packaging is performed using a commercially available in vitro packaging kit (e.g. Gigapack Gold available from PromegaBiotech), thereby producing an λ phage particles containing the recombinant λ phage DNAs. The obtained λ phage particles are transfected into host cells, e.g. E. coli, in accordance with a conventional method [Maniatis, T., et al. : Molecular Cloning. A Laboratory Manual, 2nd ed., Cold Spring Harbor Laboratory Press, 1989]. The obtained transfectants are allowed to proliferate, thereby a phage cDNA library can be obtained.

On the other hand, in regard to a group of the recombinant plasmid DNAs, for example, plasmid DNAs are transformed into host cells, e.g. E. coli, in accordance with a conventional method. The obtained transformant is allowed to proliferate, thereby a plasmid cDNA library can be obtained.

Next, the above transformants are allowed to proliferate and transferred onto a nylon membrane or a nitrocellulose membrane , e.g. Gene Screening Plus available from DuPont, and proteins are removed under a alkaline condition. The λ phage DNAs or plasmid DNAs on the membrane are hybridized with a radioactive-labeled probe derived from a partial fragment of a DNA encoding Mrell protein, then positive clones are selected. Herein, the probe to be used can be prepared by PCR using oligonucleotide primers designed based on a nucleotide sequence of a DNA encoding a partial amino acid sequence of Mrell protein. For example, for PCR-preparation of a probe to be used for screening clones including a DNA encoding yeast Mrell protein, 5'-gtgccattattatttcagaa-3' (SEQ ID NO: 9) and 5'-gggatcaagtacaactattttc-3' (SEQ ID NO: 10) may be used as a primer. Then, the obtained positive clones are cultured, and phage DNAs or plasmid DNAs are prepared from the culture.

In addition, for preparing a DNA encoding Mrell protein by direct PCR-amplification, initially genome DNA or cDNA are prepared from cells of the organisms described in section (1) above. Then, using the obtained genome DNA or cDNA as templates, a DNA encoding an amino acid sequence at N-terminal side of the Mrell protein and a DNA encoding an amino acid sequence at C-terminal side thereof are used as primers, PCR can be performed. Next, a PCR-amplified fragment is ligated to a suitable vector. Examples of primers to be used for amplifying DNA encoding yeast Mrell protein include 5'-atggactatcctgatccaga-3' (SEQ ID NO: 11) and 5'-gggatcaagtacaactattttc-3' (SEQ ID NO: 12). In the case of obtaining the DNA encoding the Mrell protein with direct PCR-amplification, pfu polymerase is preferably used as a DNA sythetase due to its infrequent misreading.

The DNA obtained according to the above method is verified to be a DNA encoding the Mrell protein through determination of a nucleotide sequence by any of known methods, e.g. the dideoxy method. Determination of a nucleotide sequence can usually be carried out by use of an automatic nucleotide sequencer (e.g. 373A DNA sequencer manufactured by PERKIN-ELMER).

In SEQ ID NOS: 1 and 3, nucleotide sequences of the DNAs encoding the Mrell proteins derived from Saccharomyces cerevisiae and human, respectively, and in SEQ ID NOS: 2 and 4, the amino acid sequences of the proteins thereof are exemplified. However, as long as the protein has physiological activity of Mrell protein, there may occur variations such as deletions, substitutions, and additions of one or more amino acids relative to these amino acid sequences.

For example, one or more, preferably about 20 to 30, more preferably 10 to 20 amino acids may be deleted from the amino acid sequence represented by SEQ ID NOS: 2 or 4. Or one or more, preferably about 20 to 30, more preferably 10 to 20 amino acids may be added to the amino acid sequence represented by SEQ ID NOS: 2 or 4. Alternatively, one or more, preferably about 20 to 30, more preferably 10 to 20 amino acids may be substituted by other amino acid(s) in the amino acid sequence represented by SEQ ID NOS: 2 or 4.

Further, a DNA which can hybridize with the above-mentioned gene under a stringent condition, as long as it encodes a protein having physiological activity of Mrell protein, can be used in the present invention. The term "a stringent condition" means that, for example, the sodium concentration is from 33 to 70 mM, preferably 50 to 66 mM and the temperature is from 40 to 70°C, preferably 55 to 68°C.

The physiological activity of Mrell protein means, as mentioned above, double-stranded DNA binding activity, double-stranded DNA exonuclease activity and single-stranded DNA endonuclease activity, and each of these activities can be determined in accordance with a method of Furuse et al. [Furuse et al. :EMBO J 17:6412-25 (1998)]

### 2. Introduction of variations into a DNA encoding foreign Mrell protein

When a DNA encoding a variant of foreign Mrell protein with modification in its nuclease domain, is introduced into a cell in a state such that the DNA can be expressed, the telomeres are shortened more than in the case where a DNA encoding a wild-type Mrell protein is introduced. However, when a DNA encoding a variant of foreign Mrell protein with modification in its C-terminal domain, is introduced in a state such that the DNA can be expressed, the telomere length is retained or increased, differing from the case where a DNA encoding a wild-type Mrell protein is introduced. Here the term "nuclease domain" means, in an amino acid sequence constituting the Mrell protein, an amino acid sequence responsible for double-stranded DNA exonuclease activity and single-stranded DNA endonuclease activity of the protein. In particular, for example with respect to yeast Mrell protein, the nuclease domain corresponds to a region from 225th to 450th amino acid numbered from the N-terminal. The term "C-terminal domain" means, in an amino acid sequence constituting the Mrell protein, an amino acid sequence at carboxyl terminal region responsible for double-stranded DNA binding activity of the protein. In particular, for example with respect to yeast Mrell protein, the C-terminal domain corresponds to a region from 600th amino acid onwards numbered from the N-terminal.

A DNA encoding a variant of Mrell protein can be prepared as follows. For example, for preparing a DNA encoding a protein having specific one or more amino acid in the nuclease domain of the Mrell protein substituted by different amino acid(s), there can be employed for example a site-directed mutagenesis method based on PCR [Saigo Kaoru et al.: "BUNSHISEIBUTSU-GAKU JIKKEN PROTOCOL I (Experiment Protocol I for Molecular Biology), p. 263 - 270, Maruzen, 1997], which is known in the technical field of the present invention.

### 3. Introduction of a DNA encoding Mrell protein or a variant of Mrell protein into a cell

A DNA encoding Mrell protein or a variant of Mrell protein can be introduced into a cell as mentioned below. That is, after preparing a recombinant vector which includes a DNA encoding the Mrell protein or variant tehreof obtained in section 1 or 2 above, the obtained recombinant vector is introduced into a host cell.

### (1) Preparation of a recombinant vector

The recombinant vector can be obtained by ligating (inserting) to a suitable vector a DNA encoding the Mrell protein or the variant of Mrell protein. Herein, the vector to be used is not particularly limited, as long as it is replicable in the host cell. Examples thereof include a plasmid vector and a virus vector.

A plasmid vector for a yeast host cell may be pMAC561aur, YEp13, YEp24, YCp50 etc., and a virus vector for an animal host cell may be a retrovirus, a vaccinia virus and the like.

For ligating the DNA encoding the Mrell protein or the variant of Mrell protein to the vector, there may be employed a method wherein initially a DNA fragment including the above DNA is cleaved by (an) appropriate restriction enzyme(s), and then it is ligated to (a) restriction site(s) or a multi cloning site in the vector DNA.

It is necessary that the DNA encoding the Mrell protein or variant of Mrell protein should be incorporated into the vector in a state such they can be expressed in the host cell. Thus, in addition to a promoter and the DNA encoding the Mrell protein or the variant of Mrell protein, a cis element such as an enhancer, a splicing signal, a poly A addition signal and a selective marker can be ligated as desired. Here, examples of the selective marker include a dihydrofolate reductase gene, a blasticidin resistant gene, an aureobasidin resistant gene, and a G418 resistant gene.

Incidentally, a recombinant vector pMACMre11D16A including a DNA encoding Mrell protein having the asparatic acid substituted by alanine at the 16th amino acid position from an N-terminal of the Mrell protein, and a recombinant vector pMACMrell ΔC49 including a DNA encoding Mrell protein having a C-terminal domain deleted each have been introduced into a E coli DH5α strain (ERKNMRE11D16AYOPAUR and ERKNMRE11DC49YOPAUR) and deposited on December 22, 1999, at the National Institute of Bioscience and Human-Technology, the National Institute of Advance Industrial Science and Technology, under the Ministry of Economy, Trade and Industry, (Higashi 1-1-3, Tsukuba, Ibaraki, Japan) under the accession Nos. FERM BP-7421 and FERM BP-7422.

### (2) Preparation of a transformant cell

Preparation of a transformant cell can be carried out by introducing the recombinant vector into a host such that a gene of interest can be expressed. Herein, the host may be, without particularly limitation, any eukaryotic cell as long as it has a filamentous chromosome including telomeres. Examples thereof include yeasts such as Saccharomyces cerevisiae and Schizosaccharomyces pombe, animal cells such as COS cells and CHO cells, or an insect cells such as Sf9, Sf21.

When using a yeast as a host, Saccharomyces cerevisiae, Schizosaccharomyces pombe, Pichia pastoris etc. may be used. In this case, any promoter may be used, without particularly limitation, that can be expressed in the yeast. Examples thereof include a gal1 promoter, a gal10 promoter, a heat shock protein promoter, a MFα1 promoter, a PH05 promoter, a PGK promoter, a GAP promoter, an ADH promoter, an AOX1 promoter. The method of introducing the recombinant vector into the yeast may be any of methods by which a DNA can be introduced into the yeast, without particular limitation, for example, electroporation [Becker, D.M. et al.:Methods. Enzymol., 194: 182(1990)], spheroplast method [Hinnen, A. et al. :Proc. Natl. Acad. Sci., USA, 75:1929(1978)], and lithium acetate method [Itoh, H. :J. Bacteriol., 153: 163 (1983)].

When using an animal cell as a host, a monkey COS-7 cell, Vero, a Chinese hamster ovary cell (CHO cell), a mouse L cell, a rat GH3, or a human FL cell can be used. As a promoter, an SRα promoter, an SV40 promoter, an LTR promoter, a CMV promoter or the like may be used, and an human cytomegalovirus early gene promoter etc. may be used as well. Exemplary methods for introducing the recombinant vector into the animal cell include electroporation, the lithium acetate method and the lipofection method.

When using an insect cell as a host, an Sf9 cell, an Sf21 cell or the like may be used. Exemplary methods for introducing the recombinant vector into the insect cell include the calcium phosphate method, the lipofection method, electroporation and the like.

### 4. Analysis of telomere length

Telomere length can be analyzed by Southern hybridization using a probe which specifically hybridizes to a telomere sequence. For example, for analyzing the telomere length in the yeast cells, initially the genome DNAs are extracted from control yeast cells and test yeast cells. Next, the obtained genome DNAs are digested by (an) appropriate restriction enzyme(s) (e.g. XhoI), and then the DNA fragments are isolated by agarose gel electrophoresis. Thereafter, they are transcribed onto a nylon membrane under an alkaline condition, and they are hybridized to a labeled-probe which specifically hybridizes with a telomere sequence under an appropriate condition. Herein, the probe may be an oligonucleotide comprising a sequence of 5'-gtgtgtgtgtgtgtgtgtgt-3' (SEQ ID NO: 13). After washing the membrane, they are visualized with autoradiography or imaging plate (e.g. BAS2000 manufactured by Fuji Photo Film Co., Ltd.). Comparison between lanes of the control yeast cells and lanes of the test yeast cells showed clearly the difference in their telomere length.

### 5. Application as an agent for controlling telomere length and a gene therapeutic agent for telomere length-related diseases

By modifying physiological activity of the endogenous Mrell protein in a eukaryotic cell, it is possible to control telomere length in the cell. Therefore, an agent comprising as an active component a substance which modifies physiological activity of endogenous Mrell protein in a eukaryotic cell, is valuable as a control agent for telomere length and a gene therapeutic agent. Examples of the substance which modifies physiological activity of endogenous Mrell protein in a eukaryotic cell, include a DNA construct comprising a DNA encoding Mrell protein or a DNA encoding a protein wherein the nuclease domain or the C-terminal domain of the Mrell protein is modified in a state such that the DNA can be expressed.

### (1) An agent for controlling telomere length

A DNA construct comprising a DNA encoding the Mrell protein a protein wherein the nuclease domain or the C-terminal domain of the Mrell protein is modified in a state such that the DNA can be expressed, is useful as a agent for controlling the telomere length (e.g. a reagent for telomere length control). That is, it is possible to control telomere length in any cell derived from any organism having a chromosome containing telomeres. To facilitate shortening of telomeres, a DNA construct comprising a DNA encoding the Mrell protein or a protein having modified a nuclease domain of the nuclease domain of the Mrell protein in a state such that either DNA can be expressed, is introduced into the cell. In contrast, to retain or increase telomere length, a DNA construct comprising a DNA encoding a protein wherein the C-terminal domain of the Mrell protein is modified in a state such that the DNA can be expressed, is introduced into the cell.

The method of introducing a gene into a yeast cell, may be any method without particular limitation as long as it is a method of introducing a DNA into a yeast, , and examples thereof include electroporation [Becker, D.M. et al. : Methods. Enzymol., 194: 182(1990)], spheroplast method [Hinnen, A. et al. : Proc. Natl. Acad. Sci., USA, 75:1929(1978)], and lithium acetate method [Itoh, H. : J. Bacteriol., 153: 163 (1983)]. Exemplary methods of introducing a gene into an animal cell include electroporation, calcium phosphate method, and lipofection method.

### (2) Gene therapeutic agent for telomere length-associated diseases

The term "telomere length-associated diseases" means any disease which relates to telomere length for the onset thereof. Examples thereof include malignant tumors (e.g. melanoma, hepatoma, breast cancer, gastric cancer, brain tumor) in which shortening of telomeres in their chromosomes does not occur because of their increased telomerase activity, and senile diseases wherein shortening of telomeres is enhanced compared with a normal cell. When a DNA construct comprising a DNA encoding the Mrell protein or a DNA encoding a protein wherein the nuclease domain or the C-terminal domain of the Mrell protein is modified in a state such that the DNA can be expressed, is used as the gene therapeutic agent for telomere length-associated diseases, the DNA construct may be directly administered to a subject by methods such as injection, gene gun etc. Examples of vector which can be used for the DNA construct, include an adenovirus vector, an adeno-associated virus vector, a herpes virus vector, a vaccinia virus vector, a retrovirus vector etc. These virus vectors enable the DNA to be more efficiently introduced into the cell. Further, a method may be employed which comprises introducing the DNA construct into a phospholipid vesicle such as liposome and administering the liposome to a subject. In this method, since the liposome is a closed vesicle including a bio-degradable material, mixing the liposome with the gene of the present invention allows the gene hold in the lipid bilayer or the internal aqueous layer of the liposome (liposome-gene complex). Next, by culturing a cell in the presence of the complexes the gene in the complex is incorporated into the cell (lipofection method). Then, the obtained cell may be administered to a subject according to administration methods described below.

As an administration form of the gene therapeutic agent of the present invention, there may be employed local administration into an epithelical tissue (epidermis) or any of various organs or tissues etc. in addition to general systemic administration e.g. intravenous or intraarterial administration etc.. Moreover, an administration form in combination with catheter technique, surgical operation etc. can be employed.

The dose of the gene therapeutic agent of the present invention is varied, depending on the age, the sex, the condition of the patient, the route or the frequency of administration, or the formulation type, but usually the range from 0.1 to 100 mg/body/day of the gene of the present invention is appropriate for adults.

### BRIEF DESCRIPTION OF THE DRAWINGS

Fig. 1 is a map of a structure of a recombinant vector.
Fig. 2 is a schematic view of an arm of an yeast chromosome.
Fig. 3 is a photograph of gel electropolation showing telomere length of a yeast cell introduced with a recombinant vector .

### BEST MODE FOR CARRYING OUT THE INVENTION

The present invention will hereinafter be described in detail with reference to Examples, but the scope of the present invention is not limited thereto.

### [EXAMPLE 1]

Preparation of a DNA recombinant vector encoding a yeast Mrell protein and a variant yeast Mrell protein

### (1) Preparation of a DNA encoding Mrell protein

E. coli cells containing a genomic DNA library of yeast DBY939 strain constructed by using a YEp24 yeast-E. coli shuttle vector (constructed by M. Carlson) were plated onto LB agar medium containing ampicillin, and the obtained colonies were transfer to a nylon membrane, then the DNA derived from the colonies onto the membrane in accordance with a conventional method. The polymerase-chain-reaction (PCR) was performed with oligonucleotides as primers, each of which has a sequence corresponding to the DNA sequence encoding the N-terminal region and the C-terminal region of the MRE11 protein respectively, and the genome DNA of wild-type blast yeast strain ORD149 as a template. The obtained DNA fragment was cloned in a vector for E. coli, and the fragment excised from the vector was labeled using [³²P]-dCTP. This probe was contacted with the nylon membrane prepared previously under stringent conditions for hybridization, thereby identifying a colony containing a fragment of Mrell gene. This colony was isolated, and the scolony hybridization was repeated similarly to above to obtain a single clone containing the Mrell gene fragment. From this plasmid DNA, the Mrell fragment was excised by restriction enzyme tdigestion and cloned into another vector compatible for E.coli, thus obtaining a plasmid pMRElltr.

### (2) Preparation of a DNA encoding a variant of Mrell protein

Using the DNA encoding the Mrell protein obtained in (1) above, in accordance with a method of Hashimoto-Gotoh et al. [Hashimoto-Gotoh, T., et al.: Gene, 152:271-275 (1995)], DNAs encoding the following proteins were prepared: a protein (also referred to as D16A type Mrell protein) wherein the C-terminal domain of the Mrell protein was deleted, a protein (also referred as to DC49 type Mrell protein) wherein the asparatic acid at 16th position from the N-terminal of the Mrell was substituted by alanine, and a protein wherein the C-terminal domain of the Mrell protein was deleted and the asparatic acid at 16th position from an N-terminal of the Mrell was substituted by alanine. That is, one nucleotide substitution from gat to get of the codon at 16th position (47th nucleotide was changed from a to c)was carried out for D16A variant, and further the 643rd and 644th codons were changed from get agt to gcc tag to introduce a termination codon, for DC49 type variant.

### (3) Construction of a recombinant vector containing a DNA encoding Mrell protein or a variant of Mrell protein

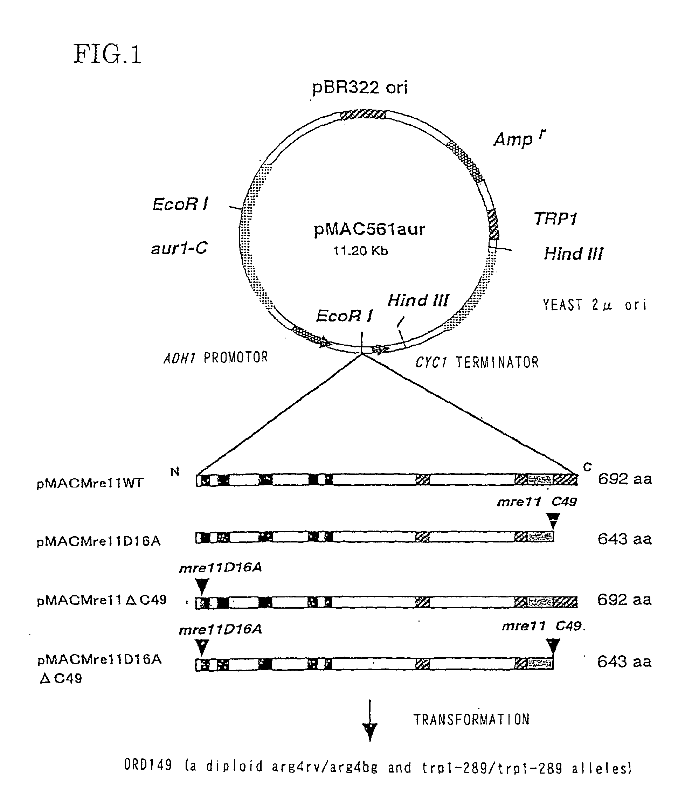

The DNA fragments obtained in (1) and (2) above which encode Mrell protein and a variant of Mrell protein, as shown in Fig. 1, were inserted at an EcoRI site present between ADH1 promoter and CYC1 terminator in a yeast-E. coli shuttle vector including an aureobasidin resistant gene, thereby constructing a vector which enables high level expression in yeast. The recombinant vector containing a DNA encoding wild type Mrell protein was named as pMACMrellWT, the recombinant vector containing a DNA encoding Mrell protein with the asparatic acid at 16th position from the N-terminal substituted by alanine was as pMACMrellD16A, the recombinant vector containing a DNA encoding Mrell protein deleted a C-terminal domain was as pMACMrellΔC49, and the recombinant vector including a DNA encoding Mrell protein wherein the asparatic acid at the 16th position from N-terminal was substituted by alanine and C-terminal domain was deletedwas as pMACMrellD16AΔC49.

### [EXAMPLE 2]

### Transformation of yeast with a recombinant vector

Transformation of yeast Saccharomyces cerevisiae ORD149 strain (a diploid having arg4rv/arg4bg and trp1-289/trp1-289 alleles), which was given by Dr. Alain Nicolas (Institut Curie, France). with the recombinant vector obtained in Example 1 was conducted, as follows. The yeast strain was washed with 1M sorbitol and mixed with the vector. After the DNA was introduced into the yeast cells by electroporation, the cells (the yeast strain having mutation in trp1 gene and the vector having a selectable marker TRP1 gene) were allowed to grow on a tryptophan-free minimal agar medium containing 1M sorbitol (, and the colonies having the plasmid were selected. These yeast colonies were further grown in a medium containing 0.2 µg/ml of aureobasidin.

### [EXAMPLE 3]

### Analysis of telomere length

The telomere length of each transformant obtained in Example 2 was analyzed as follows. That is, a yeast cell including only pMAC561aur vector and transformant yeast cells which were obtained in Example 2, each having one of the recombinant vectors, pMACMrellWT, pMACMrellD16A, pMACMrellΔC49, and pMACMrellD16AΔC49 were cultured for 12-16 hours in a nutrient medium containing 0.2 µg/ml of aureobasidin, and the cells were harvested. Next, in accordance with a conventional method, genomic DNA was extracted from the cell, and after digestion by a restriction enzyme XhoI, the DNA fragments were separated by agarose gel electrophoresis. The DNA fragments on the agarose gel were transferred onto a nylon membrane with vacuum under an alkaline condition, and the membrane was analyzed by Southern hybridization according to a method of Church et al. [Church, G.M. et. al.: Proc. Natl. Acad. Sci. U.S.A., 81: 1991-1995(1984)]. As a probe, an oligonucleotide labeled with a radioactive isotope P³² at 5'-endwhich comprises 20 nucleotides of 5'-gtgtgtgtgtgtgtgtgtgt-3' (SEQ ID NO: 13) was used. The hybridization was performed for 20 hours at 50°C. After washing, the radioactive signals on the membrane were visualized by autoradiography or with an imaging plate manufactured by Fuji Photo Film Co., Ltd.

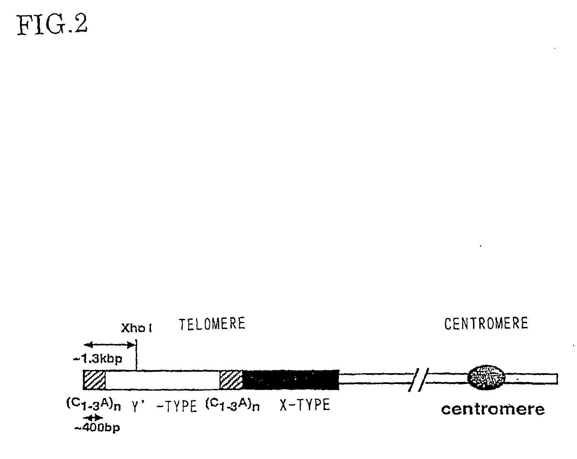

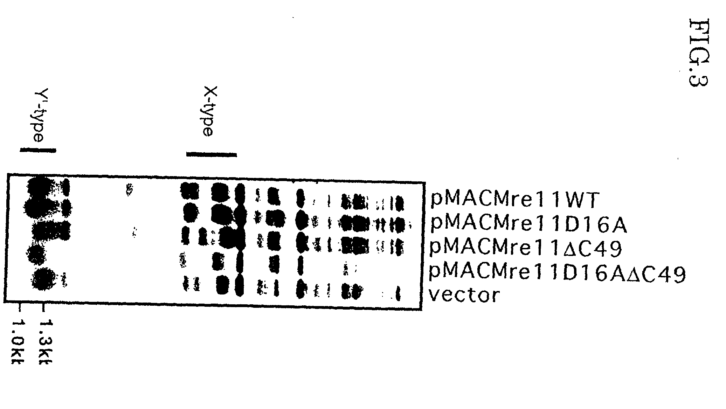

A chromosome of the obtained yeast has a structure as shown in Fig. 2. A region with which the probe can hybridize exists at some midpoints (X type) in addition to telomeres at the chromosome terminals (Y' type). In comparison with the yeast carrying only a vector not containing the DNA encoding Mrell protein, when wild type Mrell protein or the D16A type Mrell protein with a mutation in the nuclease domain was highly expressed, the shortening of telomeres in the telomere regions of both X and Y types was observed (Fig. 3) . In particular, the yeast in which the D16A type Mrell protein with a mutation in the nuclease domain was highly expressed, the shortening of telomeres was remarkable. In contrast, with regard to a cell line in which the DC49 type Mrell protein deleted the C-terminal region was highly expressed, the elongation of telomeres was observed in all telomere regions of X and Y types. In view of the forgoing, it is shown that telomeres can be elongated by introducing into a cell a DNA encoding a protein having the deletion of C-terminal domain of Mrell protein, and the telomeres can be shortened by introducinga DNA encoding Mrell protein.

### INDUSTRIAL APPLICABILITY

According to the present invention, there are provided a method of controlling telomere length, a control agent for controlling telomere length, and a gene therapeutic agent for telomere length-associated disease.

Sequence List Free Text
SEQ ID NO: 9 : synthesis DNA
SEQ ID NO: 10 : synthesis DNA
SEQ ID NO: 11 : synthesis DNA
SEQ ID NO: 12 : synthesis DNA
SEQ ID NO: 13 : synthesis DNA

## Claims

1. A method of controlling telomere length wherein the method comprises modifying physiological activity of endogenous Mrell protein in a eukaryotic cell.

2. The method of controlling telomere length according to claim 1, wherein the modification of physiological activity of the endogenous Mrell protein is carried out by introducing into a cell a DNA encoding foreign Mrell protein or a DNA encoding a protein wherein the nuclease domain or the C-terminal domain of the foreign Mrell protein is modified, in a state such that the DNA can be expressed.

3. The method of controlling telomere length according to claim 2, wherein the foreign Mrell protein is the following protein (a) or (b):
(a) a protein comprising an amino acid sequence represented by SEQ ID NO: 2 or 4; or
(b) a protein comprising an amino acid sequence which is the amino acid sequence represented by SEQ ID NO: 2 or 4 from, in, or to which one or more amino acids are deleted, substituted or added, and having physiological activity of Mrell protein.

4. The method of controlling telomere length according to claim 2, wherein the DNA encoding the foreign Mrell protein is the following DNA (c) or (d):
(c) a DNA comprising a nucleotide sequence represented by SEQ ID NO: 1 or 3; or
(d) a DNA which can hybridize with the DNA of (c) under a stringent condition and encodes a protein having physiological activity of Mrell protein.

5. The method of controlling telomere length according to claim 2, wherein the protein having the modified nuclease domain of the foreign Mrell protein is the following protein (e) or (f):
(e) a protein comprising an amino acid sequence represented by SEQ ID NO: 6; or f) a protein comprising an amino acid sequence which is the amino acid sequence represented by SEQ ID NO: 6 from, in, or to which one or more amino acids are deleted, substituted or added, and having physiological activity (except nuclease activity) of Mrell protein.

6. The method of controlling telomere length according to claim 2, wherein the DNA encoding the protein having the modified nuclease domain of the foreign Mrell protein is the following DNA (g) or (h):
(g) a DNA comprising a nucleotide sequence represented SEQ ID NO: 5; or
(h) a DNA which can hybridize with the DNA of (g) under a stringent condition and encodes a protein having physiological activity (except nuclease activity)of Mrell protein.

7. The method of controlling telomere length according to claim 2, wherein the protein having the modified C-terminal domain of the foreign Mrell protein is the following protein (i) or (j):
(i) a protein comprising an amino acid sequence represented by SEQ ID NO: 8; or
(j) a protein comprising an amino acid sequence which is the amino acid sequence represented by SEQ ID NO: 8 from, in or to which one or more amino acids are deleted, substituted or added, and having physiological activity (except double-stranded DNA binding activity) of Mrell protein.

8. The method of controlling telomere length according to claim 2, wherein the DNA encoding the protein having the modified C-terminal domain of the foreign Mrell protein is the following DNA (k) or (1):
(k) a DNA comprising a nucleotide sequence represented by SEQ ID NO: 7; or
(l) a DNA which can hybridize with the DNA of (k) and encodes a protein having physiological activity (except double-stranded DNA binding activity)of Mrell protein.

9. An agent for controlling telomere length comprising as an active component a substance which modifies physiological activity of endogenous Mrell protein in a eukaryotic cell.

10. The agent for controlling telomere length according to claim 9, wherein the substance is a DNA construct which comprises a DNA encoding a foreign Mrell protein or a DNA encoding a protein wherein the nuclease domain or the C-terminal domain of the Mrell protein is modified in a state such that the DNA can be expressed.

11. The agent for controlling telomere length according to claim 10, wherein the foreign Mrell protein is the following protein (a) or (b):
(a) a protein comprising an amino acid sequence represented by SEQ ID NO: 2 or 4; or
(b) a protein comprising an amino acid sequence which is the amino acid sequence represented by SEQ ID NO: 2 or 4 from, in, or to which one or more amino acids are deleted, substituted or added, and having physiological activity of Mrell protein.

12. The agent for controlling telomere length according to claim 10, wherein the DNA encoding the foreign Mrell protein is the following DNA (c) or (d):
(c) a DNA comprising a nucleotide sequence represented by SEQ ID NO: 1 or 3; or
(d) a DNA which can hybridize with the DNA of (c) under a stringent condition and encodes a protein having physiological activity of Mrell protein.

13. The agent for controlling telomere length according to claim 10, wherein the protein having the modified nuclease domain of the foreign Mrell protein is the following protein (e) or (f):
(e) a protein comprising an amino acid sequence represented by SEQ ID NO: 6; or
(f) a protein comprising an amino acid sequence which is the amino acid sequence represented by SEQ ID NO: 6 from, in, or to which one or more amino acids are deleted, substituted or added, and having physiological activity (except nuclease activity) of Mrell protein.

14. The agent for controlling telomere length according to claim 10, wherein the DNA encoding the protein having the modified nuclease domain of the foreign Mrell protein is the following DNA (g) or (h):
(g) a DNA comprising a nucleotide sequence represented SEQ ID NO: 5; or
(h) a DNA which can hybridize with the DNA of (g) under a stringent condition and encodes a protein having physiological activity (except nuclease activity)of Mrell protein.

15. The agent for controlling telomere length according to claim 10, wherein the protein having the modified C-terminal domain of the foreign Mrell protein is the following protein (i) or (j):
(i) a protein comprising an amino acid sequence represented by SEQ ID NO: 8; or
(j) a protein comprising an amino acid sequence which is the amino acid sequence represented by SEQ ID NO: 8 from, in or to which one or more amino acids are deleted, substituted or added, and having physiological activity (except double-stranded DNA binding activity) of Mrell protein.

16. The agent for controlling telomere length according to claim 10, wherein the DNA encoding the protein having the modified C-terminal domain of the foreign Mrell protein is the following DNA (k) or (1):
(k) a DNA comprising a nucleotide sequence represented by SEQ ID NO: 7; or
(l) a DNA which can hybridize with the DNA of (g) and encodes a protein having physiological activity (except double-stranded DNA binding activity)of Mrell protein.

17. A gene therapeutic agent for a telomere length-associated disease comprising as an active agent a substance which modifies physiological activity of endogenous Mrell protein in a eukaryotic cell.

18. The gene therapeutic agent according to claim 17, wherein the substance is a DNA construct which comprises a DNA encoding foreign Mrell protein or a DNA encoding a protein wherein the nuclease domain or the C-terminal domain of the Mrell protein is modified, in a state such that the DNA can be expressed.

19. The gene therapeutic agent according to claim 17, wherein the telomere length-associated disease is a malignant tumor or a cell senescent disease.

20. The gene therapeutic agent according to claim 19, wherein the malignant tumor is at least one selected from the group consisting of melanoma, hepatoma breast cancer, gastric cancer, and brain tumor.

21. The gene therapeutic agent according to claim 18, wherein the foreign Mrell protein is the following protein (a) or (b):
(a) a protein comprising an amino acid sequence represented by SEQ ID NO: 2 or 4; or
(b) a protein comprising an amino acid sequence which is the amino acid sequence represented by SEQ ID NO: 2 or 4 from, in, or to which one or more amino acids are deleted, substituted or added, and having physiological activity of Mrell protein.

22. The gene therapeutic agent according to claim 18, wherein the DNA encoding the foreign Mrell protein is the following DNA (c) or (d):
(c) a DNA comprising a nucleotide sequence represented by SEQ ID NO: 1 or 3; or
(d) a DNA which can hybridize with the DNA of (c) under a stringent condition and encodes a protein having physiological activity of Mrell protein.

23. The gene therapeutic agent according to claim 18, wherein the protein having the modified nuclease domain of the foreign Mrell protein is the following protein (e) or (f):
(e) a protein comprising an amino acid sequence represented by SEQ ID NO: 6; or
(f) a protein comprising an amino acid sequence which is the amino acid sequence represented by SEQ ID NO: 6 from, in, or to which one or more amino acids are deleted, substituted or added, and having physiological activity (except nuclease activity) of Mrell protein.

24. The gene therapeutic agent according to claim 18, wherein the DNA encoding the protein having the modified nuclease domain of the foreign Mrell protein is the following DNA (g) or (h):
(g) a DNA comprising a nucleotide sequence represented SEQ ID NO: 5; or
(h) a DNA which can hybridize with the DNA of (g) under a stringent condition and encodes a protein having physiological activity (except nuclease activity)of Mrell protein.

25. The gene therapeutic agent according to claim 18, wherein the protein having the modified C-terminal domain of the foreign Mrell protein is the following protein (i) or (j):
(i) a protein comprising an amino acid sequence represented by SEQ ID NO: 8; or
(j) protein comprising an amino acid sequence which is the amino acid sequence represented by SEQ ID NO: 8 from, in or to which one or more amino acids are deleted, substituted or added, and having physiological activity (except double-stranded DNA binding activity) of Mrell protein.

26. The gene therapeutic agent according to claim 18, wherein the DNA encoding the protein having the modified C-terminal domain of the foreign Mrell protein is the following DNA (k) or (1):
(k) a DNA comprising a nucleotide sequence represented by SEQ ID NO: 7; or
(l) a DNA which can hybridize with the DNA of (g) and encodes a protein having physiological activity (except double-stranded DNA binding activity)of Mrell protein.
